# EUROPEAN PATENT APPLICATION

(11) **EP 1 559 433 A1**
(43) Date of publication of application: **03.08.2005**
(21) Application number: 03756722.9
(22) Date of filing: 21.10.2003
(51) Int. Cl.: A61K 47/18, A61K 9/20, A61K 47/26, A61K 47/42, A61K 47/36

(54) **STABILIZED COMPOSITION**

(30) Priority: 22.10.2002 JP 2002307241; 30.06.2003 JP 2003186591
(71) Applicant: Dainippon Pharmaceutical Co., Ltd., Osaka-shi, Osaka 541-8524 (JP); Takeda Pharmaceutical Company Limited, Osaka 541-0045 (JP)
(72) Inventor: IWATA, Motokazu c/o Dainippon Pharm.Co. Ltd., Osaka-shi, Osaka 553-0001 (JP); FUJITA, Megumi c/o Dainippon Pharm.Co. Ltd., Osaka-shi, Osaka 553-0001 (JP); KURITA, Hideo c/o Dainippon Pharm.Co. Ltd., Osaka-shi, Osaka 553-0001 (JP); KIYOSHIMA, Kenichiro Takeda Pharmaceutical Co.Ltd., Osaka-shi, Osaka 532-8686 (JP)
(74) Representative: Coleiro, Raymond
(86) International application number: PCT/JP2003/013419
(87) International publication number: WO 2004/037293

(57) **Abstract**

The present invention provides a composition containing a substance being capable of supplying aldehyde-like substances to be used in the field of medicaments, cosmetics, hair care products, etc., that contains a low-molecular weight active substance the stability of which is impaired by the effects of aldehydes and a stabilizer having an amine structure and being capable of absorbing aldehydes, and has an improved stability to aldehyde of said low-molecular weight active the stability of which is impaired by the effects of aldehydes substance; and a method for stabilizing such a low-molecular weight active substance. An example of said stabilizer is an aminosugar or a polymer thereof, an aminosugar alcohol or a polymer thereof, an amino acid or a polymer thereof, a protein or a hydrolysate thereof, an alkylamine, a hydroxyalkylamine, or a salt thereof.

## Description

### TECHNICAL FIELD

The present invention relates to a composition containing a substance being capable of supplying aldehyde-like substances, which comprises a low-molecular weight active substance the stability of which is impaired by the effects of aldehydes, and a stabilizer having an amine structure and being capable of absorbing aldehydes (hereinafter, occasionally simply referred to as the composition of the present invention), and a method for stabilizing said low-molecular weight active substance.

### BACKGROUND ART

In compositions for medicaments, cosmetics, hair care products, etc., it is very important to safely retain effective activities of a low-molecular weight active substance contained therein. When selecting other additives to be added to compositions, it is necessary to study the reactivity thereof with a low-molecular weight active substance contained in said compositions. For example, JP-A-10-502355 discloses a topical composition containing N-acetyl-L-cysteine, which is substantially free of formaldehyde in order to solve a problem that formaldehyde chemically reacts with N-acetyl-L-cysteine to decrease its activity.

It has been known that compounds having a primary amine or a secondary amine within the structure thereof may easily react with formaldehyde or a compound having an aldehyde group to form an imine or enamine, which is a reversible reaction intermediate (cf., Stanley H. Pine, ORGANIC CHEMISTRY, 5th edition, pp. 248-251 (1987), McGRAW-HILL BOOK COMPANY, New York), and it is believed that a further irreversible reaction may proceed to form a related compound depending on structures of intermediates.

On the other hand, it has been known that a solid pharmaceutical composition or various additives to be contained therein may slightly contain formaldehyde or other aldehyde-like substances. It has also been known that formaldehyde or other aldehyde-like substances are generated when the additives or solid pharmaceutical compositions are stored under conditions of high temperature and high humidity (cf., Pharmaceutical Research, 1998, vol. 15, no. 7, p. 1026-1030; and Journal of Pharmaceutical Sciences, 1994, vol. 83, no. 7, p. 915-921).

Therefore, when adding a low-molecular weight active substance the stability of which is impaired by the effects of aldehydes into such compositions, it is necessary to stabilize said active substance.

JP-A-3-41033 discloses a motilin compound-containing lyophilized pharmaceutical composition, which comprises a motilin compound and a stabilizer selected from pharmaceutically acceptable saccharides, amino acids, inorganic salts and proteins. However, this patent publication discloses merely a stabilization method in a lyophilized pharmaceutical composition of a high-molecular weight substance, and it does not mention effects of aldehydes.

WO 02/064133 publication discloses a preparation comprising one or more components selected from alkaline agents, amino acids and gelatin, and [3-[(2R)-[[(2R)-(3-chlorophenyl)-2-hydroxyethyl]amino]propyl]-1H-indol-7-yloxy]acetic acid or a lower alkyl ester thereof, and the data of stability test of an aqueous solution thereof with adjusting the pH value with an alkaline agent is disclosed. However, this publication never discloses the relationship with aldehydes.

### DISCLOSURE OF INVENTION

An object of the present invention is to provide a composition containing a substance being capable of supplying aldehyde-like substances to be used in the field of medicaments, cosmetics, hair care products, etc., wherein the stability to aldehydes of a low-molecular weight active substance the stability of which is impaired by the effects of aldehydes is improved.

According to the study of the present inventors, it has been found that a composition containing a low-molecular weight active substance the stability of which is impaired by effects of aldehyde has a defect that said active substance is decomposed by a substance being capable of supplying aldehyde-like substances to be added to said composition, for example, by a substance such as an aldehyde group-containing excipient, etc. during the production process of said composition or in the storage thereof so that the activity of said active substance is decreased. They have intensively studied in order to find a method for preventing such decrease in activity, and found that the decrease in activity of said active substance may effectively be suppressed by adding a compound having an amine structure and being capable of absorbing aldehydes as a stabilizer, and they have accomplished the present invention.

Namely, the present invention provides compositions and stabilization methods as shown in the following embodiments.
[1] A composition containing a substance being capable of supplying aldehyde-like substances, which further comprises a low-molecular weight active substance the stability of which is impaired by the effects of aldehydes, and a stabilizer having an amine structure and being capable of absorbing aldehydes.
[2] The composition according to the above [1], wherein the stabilizer is an aminosugar or a polymer thereof, an aminosugar alcohol or a polymer thereof, an amino acid or a polymer thereof, a protein or a hydrolysate thereof, an alkylamine, a hydroxyalkylamine, or a salt thereof.
[3] The composition according to the above [2], wherein the stabilizer is chitin, chitosan, chitooligosaccharide, meglumine, alanine, arginine, lysine, hydroxylysine, gelatin or a hydrolysate thereof, collagen or a hydrolysate thereof, albumin or a hydrolysate thereof, casein or a hydrolysate thereof, protamine or a hydrolysate thereof, diethylamine, hexylamine, tris(hydroxymethyl)aminomethane, or a salt thereof.
[4] The composition according to the above [3], wherein the stabilizer is meglumine, or a salt thereof.
[5] The composition according to any one of the above [1]-[4], which is a pharmaceutical composition containing a low-molecular weight active substance and a stabilizer both in the form of a solid powder.
[6] The composition according to the above [5], which is a pharmaceutical composition of solid form or semisolid form.
[7] The composition according to the above [6], which is the solid or semisolid pharmaceutical composition selected from powders, fine granules, granules, tablets, capsules, powdery injections, dry powder inhales (inhalants, inhalations), ointments, and adhesive preparations.
[8] The composition according to the above [1], which is prepared by uniformly mixing a low-molecular weight active substance and a stabilizer.
[9] The composition according to the above [1], which is prepared by previously granulating one of a low-molecular weight active substance and a stabilizer together with a substance being capable of supplying aldehyde-like substances, followed by uniformly mixing the resultant with the other.
[10] The composition according to the above [9], which is prepared by previously granulating a stabilizer together with a substance being capable of supplying aldehyde-like substances, followed by uniformly mixing the resultant with a low-molecular weight active substance so that the contact between the substance being capable of supplying aldehyde-like substances and the low-molecular weight active substance is prevented or lessened.
[11] A pharmaceutical composition, which comprises a mass containing a low-molecular weight active substance the stability of which is impaired by the effects of aldehydes, and a mass containing a stabilizer having an amine structure and being capable of absorbing aldehydes, and at least one of these masses contains a substance being capable of supplying aldehyde-like substances.
[12] The composition according to the above [11], wherein both of the mass containing a low-molecular weight active substance and the mass containing a stabilizer are in the form of a granule.
[13] The composition according to the above [11], wherein both of the mass containing a low-molecular weight active substance and the mass containing a stabilizer are in the form of a fine granule.
[14] The composition according to the above [11], which is in the form of capsules prepared by filling granules and/or fine granules containing a low-molecular weight active substance, and granules and/or fine granules containing a stabilizer into capsules.
[15] The composition according to the above [11], which is in the form of tablets prepared by tableting granules and/or fine granules containing a low-molecular weight active substance, and granules and/or fine granules containing a stabilizer.
[16] A method of stabilizing a low-molecular weight active substance the stability of which is impaired by the effects of aldehydes in a composition containing a substance supplying aldehyde-like substances, which comprises adding a stabilizer having an amine structure and being capable of absorbing aldehydes when mixing said low-molecular weight active substance the stability of which is impaired by the effects of aldehydes.
[17] The stabilization method according to the above [16], which comprises uniformly mixing a substance supplying aldehyde-like substances, a low-molecular weight active substance the stability of which is impaired by the effects of aldehydes and a stabilizer having an amine structure and being capable of absorbing an aldehyde.
[18] The stabilization method according to the above [16], which comprises previously granulating one of a low-molecular weight active substance the stability of which is impaired by the effects of aldehydes and a stabilizer having an amine structure and being capable of absorbing aldehydes together with a substance supplying aldehyde-like substances, followed by mixing the resultant with the other.
[19] The stabilization method according to the above [18], which comprises previously granulating a stabilizer having an amine structure and being capable of absorbing aldehydes together with a substance supplying aldehyde-like substances, followed by mixing the resultant with a low-molecular weight active substance the stability of which is impaired by the effects of aldehydes.
[20] The stabilization method according to the above [16], which comprises preparing a mass containing a low-molecular weight active substance the stability of which is impaired by the effects of aldehydes and a mass containing a stabilizer having an amine structure and being capable of absorbing aldehydes separately, during which a substance supplying aldehyde-like substances is contained in one or both of these messes, followed by combining and mixing these two masses.

### BRIEF DESCRIPTION OF DRAWINGS

Fig. 1 is a view showing a stoppered glass test tube for confirming the generation of aldehydes.
Fig. 2 is a view showing a stoppered glass test tube for confirming the reaction between a low-molecular weight active substance and aldehydes.
Fig. 3 is a view showing a stoppered glass test tube for confirming the reaction between a low-molecular weight active substance and aldehydes within a pharmaceutical composition.
Fig. 4 is a view showing a stoppered glass test tube for confirming the absorption effect of aldehyde by a stabilizer.

### Explanation of symbols:

- 1: Stoppered glass test tube
- 2: Purified water
- 3: Support rod
- 4: Solid pharmaceutical composition
- 5: Aqueous formaldehyde solution
- 6: Power of Compound A
- 7: Tablets containing Compound A
- 8: Meglumine

### BEST MODE FOR CARRYING OUT THE INVENTION

The composition of the present invention is a composition containing a substance being capable of supplying aldehyde-like substances and a low-molecular weight active substance the stability of which is impaired by the effects of aldehydes, where by adding a stabilizer having an amine structure and being capable of absorbing aldehydes, the effect of said substance being capable of supplying aldehyde-like substances on said active substance are suppressed and the decrease in activity thereof is well prevented.

The composition of the present invention may be any composition for medicaments, cosmetics, hair care products, etc.

In the present invention, the "substance being capable of supplying aldehyde-like substances" may be any possible substance which may supply "aldehyde impairing the stability of low-molecular weight active substances" as mentioned below, and it may be either a substance having an aldehyde structure or a substance being capable of producing or generating a compound having an aldehyde structure during the production process of the composition or during the storage. The "substance being capable of supplying aldehyde-like substances" may be a substance being capable of producing or generating a compound having an aldehyde structure, or a substance being capable of producing or generating a compound having an aldehyde structure by interaction with other components in the above-mentioned composition or with environmental factors (e.g., moisture, oxygen, carbon dioxide). Examples of the "substance being capable of supplying aldehyde-like substances" are compounds having an aldehyde structure as mentioned below (e.g., formaldehyde, acetaldehyde, propionaldehyde, n-butylaldehyde, isobutylaldehyde, n-valeraldehyde, isovaleraldehyde), a carbohydrate having an aldehyde structure within the structure thereof (e.g., aldose such as glucose, galactose, mannose, xylose, etc.), an additive which is converted by hydrolysis into a carbohydrate having an aldehyde group within the structure thereof (e.g., oligosaccharide such as lactose, sucrose, trehalose, etc., polysaccharide such as starch, cellulose, etc.), an additive which is converted by oxidization into a carbohydrate having an aldehyde structure within the structure thereof (e.g., sugar alcohol such as mannitol, sorbitol, xylitol, erythritol, etc.), and further a lower alkyl alcohol such as methyl alcohol, ethyl alcohol, etc., a polyethylene alcohol or a fatty acid ester thereof, and polyethylene glycol or a fatty acid ester, etc.

The "low-molecular weight active substance the stability of which is impaired by the effects of aldehydes" of the present invention (hereinafter, occasionally referred to as low-molecular weight active substance) may be any low-molecular weight substance exhibiting some kind of useful effects, e.g., a pharmacological activity, and producing a related substance by reacting with aldehydes within the composition of the present invention. It may be determined, for example, by the method of Experiment 2 as mentioned below, if a low-molecular weight active substance can produce a related substance by reacting with aldehyde or not. The low-molecular weight active substance also includes, for example, compounds having a primary amine structure or a secondary amine structure, compounds having a hyrazine structure or a tryptamine structure. In case of pharmaceutical compositions, examples of the low-molecular weight active substance are dopamine, methyldopa, norepinephrine, baclofen, hydralazine, epinephrine, isoproterenol, nadolol, tulobuterol, ephedrine, phenylephrine, etilefrine, propranolol, [3-[(2R)-[[(2R)-(3-chlorophenyl)-2-hydroxyethyl]amino]propyl]-1H-indol-7-yloxy]acetic acid, an ester thereof, or a salt thereof.

In the present invention, the "aldehyde impairing the stability of low-molecular weight active substance" means a compound having an aldehyde structure, and includes, for example, formaldehyde, acetaldehyde, propionaldehyde, n-butylaldehyde, isobutylaldehyde, n-valeraldehyde, isovaleraldehyde. Carbohydrates having an aldehyde group within the structure thereof, for example, aldose such as glucose, galactose, mannose, xylose, etc. may also be included therein. The aldehydes may be supplied within compositions either in the case where a compound having an aldehyde structure as mentioned above per se exists in said compositions or in the case where a substance being capable of producing or generating a compound having an aldehyde structure exists in said compositions. Namely, the "aldehyde impairing the stability of low-molecular weight active substance" may be ones produced or generated by interaction with other components in the composition or with environmental factors (e.g., moisture, oxygen, carbon dioxide). The additives producing or generating a compound having an aldehyde structure within the composition may be, for example, oligosaccharides such as lactose, sucrose, trehalose, etc., polysaccharides such as starch, cellulose, etc., which is converted by hydrolysis into a carbohydrate having an aldehyde group within the structure thereof. Further, sugar alcohols such as mannitol, sorbitol, xylitol, erythritol, etc. which may converted by oxidization into a carbohydrate having an aldehyde group within the structure thereof may also be included. In addition, a lower alkyl alcohol such as methyl alcohol, ethyl alcohol, etc., polylethylene alcohol or a fatty acid ester thereof, and polylethylene glycol or a fatty acid ester thereof may also be additives producing or generating a compound having an aldehyde group within the composition.

The "stabilizer having an amine structure and being capable of absorbing aldehydes" used in the present invention (hereinafter, occasionally referred simply to as the stabilizer) means a stabilizer simultaneously having the following two features: (1) having an amine structure, as well as (2) being capable of absorbing aldehydes. It may be confirmed by the method of Experiment 4 as mentioned below whether the stabilizer may absorb aldehydes or not. Further, the stabilizer should have a safety enough to be accepted in the field of each composition. Examples thereof are an amino acid or a polymer thereof, an aminosugar alcohol or a polymer thereof, an amino acid or a polymer thereof, a protein or a hydrolysate thereof, an alkylamine, a hydroxyalkylamine, or a salt thereof. These stabilizers may be used alone or in a combination of two or more of these stabilizers.

The "aminosugar" includes, for example, N-acetylgalactosamine, N-acetylglucosamine, galactosamine, and glucosamine. A salt of an aminosugar, a polymer of aminosugar or a salt thereof may be employed. The "polymer of aminosugar" may be, for example, chitin, chitosane, chitoligosaccharide, etc.

The "aminosugar alcohol" means a sugar alcohol having an amino group, and includes, for example, meglumine. A salt of aminosugar alcohol, a polymer of aminosugar alcohol or a salt thereof may be used.

The "amino acid" includes, for example, basic amino acids, more particularly, alanine, arginine, lysine, hydroxylysine, ornithine, etc. Besides, the "amino acid" also includes glutamine, aspartic acid, citrulline, etc., and a salt of an amino acid, a polymer of an amino acid or a salt thereof may also be used. The "polymer of an amino acid" is, for example, polylysine.

The "protein" includes, for example, gelatin, collagen, albumin, casein, protamine, and a salt of protein may also be used. Further, a hydrolysate of protein may also be used.

The "alkylamine" is a straight chain or branched chain alkylamine having 1 to 6 carbon atoms, for example, diethylamine, hexylamine, or a salt thereof. The "hydroxyalkylamine" is the above alkylamine being substituted by 1 to 3 hydroxy groups, for example, tris(hydroxymethyl)-aminomethane or a salt thereof.

As a stabilizer, an anion exchange resin having an amino group as a functional group (e.g., cholestyramine (trade name; Amberite IRP43), DEAE-cellulose, DEAE-agalose, meglumine-cellulose), etc. may be used.

Preferable stabilizer to be used in the present invention includes, for example, meglumine, tris(hydroxymethyl)aminomethane, L-arginine, gelatin, or a salt thereof.

In the above low-molecular weight active substance and the stabilizer, the "salt" means an acid addition salt, an alkali metal salt, an alkaline earth metal salt or a salt with an organic base. Examples of the acid addition salt are a salt with an inorganic acid such as hydrochloride, hydrobromide, hydriodate, sulfate, phosphate, etc., or a salt with an organic acid such as oxalate, maleate, fumarate, malonate, lactate, malate, citrate, tartrate, benzoate, methanesulfonate, p-toluenesulfonate, gluconate, etc. The alkali metal salt includes, for example, a salt with an inorganic alkali metal such as sodium salt, potassium salt, etc., and the alkaline earth metal salt includes, for example, calcium salt, magnesium salt, and the salt with an organic base includes, for example, a salt with ammonia, methylamine, triethylamine, tributylamine, diisopropylethylamine, N-methylmorpholine or dicyclohexylamine.

The stabilizer to be used in the composition of the present invention should be selected in view of the weight and the reactivity of the substance being capable of supplying aldehyde-like substances existing within the composition, the amount of aldehyde-like substances being supplied in the composition, the weight, the molecular weight and the reactivity of the low-molecular weight active substance to aldehydes, and the reactivity of the stabilizer to aldehydes, and it is preferable to select a stabilizer having a higher reactivity than that of the low-molecular weight active substance to aldehydes.

The amount of the stabilizer to be added to the composition of the present invention should preferably be selected according to the weight of the substance being capable of supplying aldehyde-like substances, the amount of the aldehyde being supplied within the composition, the reactivity of the low-molecular weight active substance to aldehydes, the weight and the molecular weight of the low-molecular weight active substance, the total weight of the composition, and the other characteristics.

In one embodiment of the present invention, the composition of the present invention is prepared by uniformly mixing a low-molecular weight active substance and a stabilizer with a substance being capable of supplying aldehyde-like substances. Namely, a low-molecular weight active substance and a stabilizer are simultaneously or successively added to a substance being capable of supplying aldehyde-like substances, and if necessary, other additives are added to the resultant, and the mixture is mixed or kneaded to give a uniform composition.

In another embodiment of the present invention, the composition of the present invention may be prepared by previously granulating one of a low-molecular weight active substance and a stabilizer together with a substance being capable of supplying aldehyde-like substances, then followed by uniformly mixing the resulting with the other. In a more preferable embodiment, a stabilizer is previously granulated together with a substance being capable of supplying aldehyde-like substances, and then the resultant is uniformly mixed with a low-molecular weight active substance. In this process, the contact between a substance being capable of supplying aldehyde-like substances and a low-molecular weight active substance is prevented or decreased, and hence, there is obtained a composition wherein said low-molecular weight active substance is more stably retained.

In further embodiment of the present invention, the composition of the present invention is prepared by separately preparing a mass containing a low-molecular weight active substance and a mass containing a stabilizer during which a substance being capable of supplying aldehyde-like substances is contained in one of these masses, then followed by mixing or kneading these two masses.

In the present specification, the "mass" means that it contains a low-molecular weight active substance or a stabilizer, if necessary, further contains a substance being capable of supplying aldehyde-like substances and/or other additives, and after mixing these masses to formulate into a composition, it constitutes a region where said low-molecular weight active substance or said stabilizer is retained in a comparatively high concentration, respectively.

More particularly, the "mass" means granules or fine granules which are prepared by mixing or kneading a low-molecular weight active substance or a stabilizer together with a substance being capable of supplying aldehyde-like substances and/or other additives if required. Then, the granules or fine granules may be any ones as long as they are granulated substances (a granulation method thereof may be either dry granulation or wet granulation), but the granules or fine granules of the pharmaceutical composition of the present invention preferably have a particle size specified by The Japanese Pharmacopoeia Fourteenth Edition. Namely, the granules mean preparations where all the granules pass through a No. 10 (1700 µm) sieve, not more than 5 % of total granules remain on a No. 12 (1400 µm) sieve, and not more than 15 % of total granules pass through a No. 42 (355 µm) sieve when the particle size distribution test is performed with granules. On the other hand, the fine granules are preparations where when the Particle Size Distribution Test is performed, all the fine granules pass through a No. 18 (850 µm) sieve and not more than 5 % of total fine granules remain on a No. 30 (500 µm) sieve, and not more than 10 % of total fine granules pass through a No. 200 (75 µm) sieve.

The average particle size of granules or fine granules is, for example, in the range of 1 µm to 5 mm, preferably in the range of 10 µm to 1 mm, more preferably in the range of 20 µm to 500 µm.

The above-mentioned "mass" may be granules or fine granules per se, or may be ones which are obtained by physically pulverizing granules or fine granules during the production process of pharmaceutical compositions.

Namely, the "mass" may be granules or fine granules retaining an original form, for example, ones being contained in capsules, etc., or a region which does not retain an original form by physically pulverizing during the tableting process but contains a low-molecular weight active substance or a stabilizer in a high concentration, respectively. Further, the "mass" may be a core constituting core-shell granules, or a region being adhered to core-shell granules (including partially or wholly coating).

The composition of the present invention includes a composition to be used in various uses such as medicaments, cosmetics, hair care products, etc., and most important ones are pharmaceutical compositions.

The pharmaceutical composition is explained in more detail below.

The pharmaceutical composition of the present invention is a composition containing a low-molecular weight active substance and a stabilizer, both in the form of a solid powder, and preferably a pharmaceutical composition in the form of a solid or semisolid preparation. Examples of the pharmaceutical composition are powders, fine granules, granules, tablets, capsules, powdery injections, dry powder inhales, ointments or adhesive preparations. The above-mentioned capsules include hard capsules or soft capsules containing powders, fine granules, granules, tablets, ointments. The granules and fine granules mean granules and fine granules having a particle size specified by The Japanese Pharmacopoeia Fourteenth Edition.

The pharmaceutical composition consisting of a mass containing a low-molecular weight active substance and a mass containing a stabilizer, and a substance being capable of supplying aldehyde-like substances being added to at least one of these masses is preferably a solid pharmaceutical composition, and examples thereof are granules, fine granules, tablets and capsules, and especially tablets and capsules are preferable. Then, the tablets may include intraorally disintegrating tablets, chewable tablets, sublingual tablets, etc. Further, the pharmaceutical composition may be a preparation which is dissolved when use.

The low-molecular weight active substance and the stabilizer to be used in the pharmaceutical composition of the present invention are in the form of a solid powder (crystalline or noncrystalline form), which may be used as they stand, or may be used after pulverizing with a suitable mill, for example, fluid energy mill, a hammer mill, a ball mill, a vibratory ball mill, a planetary ball mill, etc. to adjust the average particle size thereof into not larger than 200 µm. Preferably, the low-molecular weight active substance and the stabilizer are preferably used after granulating into ones having the average particle size of 100 µm or below, more preferably ones having the average particle size of 50 µm or below. Further, they may be used after suitably adjusting the particle size thereof by suitably adjusting the crystallization conditions in the final stage of the production or by using a supercritical fluid technique without the pulverization process.

As the stabilizer to be used in the pharmaceutical composition of the present invention, it is preferable to select one having a higher reactivity to aldehydes than that of the low-molecular weight active substance.

In the pharmaceutical composition of the present invention, the amount of the stabilizer may vary according to the reactivity of a low-molecular weight active substance to aldehydes, the molecular weight of a low-molecular weight active substance, and other characteristics, and the stabilizer is usually added in an amount of 0.001 1 to 1000 parts by weight to one part by weight of the low-molecular weight active substance in the pharmaceutical composition. Preferably, the stabilizer is added in an amount of 0.01 to 100 parts by weight to one part by weight of the low-molecular weight active substance, and more preferably it is added in an amount of 0.1 to 50 parts by weight to one part by weight of the low-molecular weight active substance. Most preferably, it is added in an amount of 0.2 to 20 parts by weight to one part by weight of the low-molecular weight active substance.

In addition, in the pharmaceutical composition consisting of a mass containing a low-molecular weight active substance and a mass containing a stabilizer, the ratio of the low-molecular weight active substance-containing mass and the stabilizer-containing mass is in the range of 0.01:100 to 100:0.01 (by weight, hereinafter the same), preferably in the range of 0.1:50 to 50:0.1, more preferably in the range of 0.1:10 to 10:0.1, converting into the weight of the low-molecular weight active substance and the stabilizer, respectively.

Further, in light of the fact that aldehydes generated within the pharmaceutical composition from a substance being capable of supplying aldehyde-like substances affect on the chemical stability of a low-molecular weight active substance, the amount of the stabilizer to be added should be determined according to the total weight of the substance being capable of supplying aldehyde-like substances within the pharmaceutical composition. For example, the stabilizer is added in an amount of 0.0001 to 0.5 part by weight, preferably in an amount of 0.001 to 0.1 part by weight, more preferably in an amount of 0.005 to 0.05 part by weight, based on the total weight of the substance being capable of supplying aldehyde-like substances.

Although depending on the property of a substance being capable of supplying aldehyde-like substances existing in the pharmaceutical composition, the stabilizer is usually added at a ratio of 0.0001 to 0.5 part by weight, based on the total weight of the pharmaceutical composition. Preferably, the stabilizer is added at a ratio of 0.001 to 0.1 part by weight, based on the total weight of the pharmaceutical composition. More particularly, the stabilizer is added at a ratio of 0.005 to 0.05 part by weight, based on the total weight of the pharmaceutical composition.

As mentioned above, the amount of the stabilizer to be added to the pharmaceutical composition of the present invention may be decided based on either the weight of the low-molecular weight active substance in the pharmaceutical composition, the weight of the substance being capable of supplying aldehyde-like substances in the pharmaceutical composition, or the total weight of the pharmaceutical composition, but in either case, it is preferable to select the amount being most suitable for stabilizing the low-molecular weight active substance in the pharmaceutical composition.

The production of the pharmaceutical composition of the present invention is conducted, for example, in the following manner. In an embodiment of the present invention, the pharmaceutical composition of the present invention is prepared by uniformly mixing a low-molecular weight active substance and a stabilizer together with a substance being capable of supplying aldehyde-like substances. For example, a prescribed amount of low-molecular weight active substance and a prescribed amount of stabilizer are added simultaneously or successively to a pharmaceutical carrier containing a substance being capable of supplying aldehyde-like substances in a mixer, and if necessary, other additives are added thereto, and the resultant is uniformly mixed to give powders. In order to prepare fine granules or granules, a wetting agent, a binder, a disintegrant, etc. is further added thereto, and the resultant is subjected to steps such as kneading, granulating, drying, regulating in size, sieving, etc. to produce a commercialized product of the pharmaceutical composition of the present invention.

In other embodiment of the present invention, one of a low-molecular weight active substance and a stabilizer is previously granulated together with a substance being capable of supplying aldehyde-like substances, and the resultant is uniformly mixed with the other one to give the pharmaceutical composition of the present invention. More preferably, the present pharmaceutical composition is prepared by previously granulating a stabilizer together with a substance being capable of supplying aldehyde-like substances, followed by uniformly mixing the resultant with a low-molecular weight active substance so that the contact between a substance being capable of supplying aldehyde-like substances and a low-molecular weight active substance is prevented or decreased as low as possible. Hereinafter, the process where a low-molecular weight active substance is previously granulated with a substance being capable of supplying aldehyde-like substances and the resultant is uniformly mixed with a stabilizer is expressed as the stabilizer-post-addition method, and the process where a stabilizer is previously granulated with a substance being capable of supplying aldehyde-like substances and the resultant is uniformly mixed with a low-molecular weight active substance is occasionally expressed as the low-molecular weight active substance post-addition method.

In addition, when the pharmaceutical composition of the present invention is granules, tablets or capsules, then one of a stabilizer and a low-molecular weight active substance is granulated together with a substance being capable of supplying aldehyde-like substances to give granules (core particulate) or tablets (core tablet), then the other one is added to the resulting granules or tablets as a component consisting of a coating agent such as sugar coat, film coat or a component forming capsules.

In further embodiment of the present invention, a mass containing a low-molecular weight active substance and a mass containing a stabilizer are separately prepared, during which a substance being capable of supplying aldehyde-like substances is added to one or both of these masses, and then both masses are mixed and formulated to give the pharmaceutical composition of the present invention.

For example, a low-molecular weight active substance and a stabilizer are previously granulated separately during which a substance being capable of supplying aldehyde-like substances is added to one or both of these granules, and then, both granules are mixed or kneaded to give pharmaceutical compositions. The granules containing a low-molecular weight active substance or a stabilizer may be core-shell granules which may be prepared by adhering (including partially or wholly coating) a low-molecular weight active substance or a stabilizer to a substantially spherical core such as Nonpareil (trade name; consisting of sucrose 75 % (w/w) and corn starch 25 % (w/w)). Hereinafter, the process as mentioned above where a low-molecular weight active substance and a stabilizer are granulated separately into granules or fine granules, and formulated to give pharmaceutical compositions is occasionally expressed as the two-group granulation method.

Further, the substance being capable of supplying aldehyde-like substances is preferably added to both of a mass (preferably granules) containing a low-molecular weight active substance or a mass (preferably granules) containing a stabilizer, or only into granules containing a stabilizer.

The low-molecular weight active substance-containing granules and the stabilizer-containing granules are put into capsules as they stand to give capsules, or a mixture of these granules is tableted by a conventional method to give tablets. Further, the low-molecular weight active substance-containing granules and the stabilizer-containing granules may be uniformly distributed in a composition, for example, these granules may exist individually in a discrete part of formulations such as multilayer tablets or core-shell tablets.

In addition, a low-molecular weight active substance or a stabilizer is successively adhered (including partially or wholly coating) or laminated, if necessary, together with a substance being capable of supplying aldehyde-like substances, onto a substantially spherical core such as Nonpareil (trade name; consisting of sucrose 75 % (w/w) and corn starch 25 % (w/w)), etc. to give pharmaceutical compositions. Moreover, one of a low-molecular weight active substance and a stabilizer is regarded as a core, and the other one is adhered (including partially or wholly coating) or laminated onto the core, if necessary, together with a substance being capable of supplying aldehyde-like substances, to give pharmaceutical compositions.

The core-shell granules containing a low-molecular weight active substance and a stabilizer thus obtained may be put into capsules as they stand to give capsules, or said core-shell granules are tableted by a conventional method to give tablets.

Further, when the compatibility between a low-molecular weight active substance and a stabilizer is not good, then among the above-mentioned processes, the pharmaceutical composition wherein said low-molecular weight active substance is most stabilized is obtained by employing the above-mentioned two-group granulation method. Namely, in the pharmaceutical composition obtained by the two-group granulation method, as compared to other pharmaceutical compositions obtained by other various processes, the decrease in activity of the low-molecular weight active substance or the generation of related substances thereof is suppressed.

The pharmaceutical composition of the present invention thus obtained shows a good stability when stored by any forms such as heat-seal package, press-through package or bottle package, etc. A better stability is obtained by moisture-proof secondary packaging such as aluminum pillow, etc. in case of heat-seal package or press-through package, or by putting into a high-density polyethylene bottle, a glass bottle, etc. in cases of bottle package. Further, by enclosing silica gel into a moisture-proof secondary package or a bottle, the best stability is obtained.

In the above-mentioned packages or bottles, one or more of desiccants, deoxidizers, carbon dioxide absorbents, moisture adjusters, etc. may additionally be enclosed, if necessary, after they are packed in a canister.

For example, the desiccant includes silica gel, calcium oxide, calcium chloride products, silica-alumina gel, synthesized zeolite, diatomite, etc.; the deoxidizer includes metal powders such as iron powder, etc. (including a metal halide as an oxidizing catalyst), a sulfite salt, a bisulfite salt, dithionous acid, hydroquinone, catechol, resorcin, pyrogallol, gallic acid, rongalit, ascorbic acid, ascorbate, isoascorbic acid, isoascorbate, sorbose, glucose, lignin, etc.; the carbon dioxide absorbent includes calcium hydroxide, magnesium hydroxide, calcium oxide, magnesium oxide, soda lime, bara lime, calcium silicate hydrate, etc.; the moisture adjuster includes polyethylene glycol, propylene glycol, inorganic salts, type-B silica gel, paper, cellulose, charcoal, a strip being impregnated with a saturated aqueous solution of an inorganic salt. They are used in an amount conventionally used in the package design for medicaments.

Each formulation of the pharmaceutical composition of the present invention is illustrated in more detail. For convenience, the compositions of the present invention is illustrated with grouping into an embodiment which is formulated by uniformly mixing a low-molecular weight active substance and a stabilizer (including an embodiment which is formulated by granulating one of them in advance, followed by uniformly mixing the resultant with the other one), and an embodiment which is formulated by separately preparing a mass containing a low-molecular weight active substance and a mass containing a stabilizer, followed by using both masses.

### (I) In cases where a low-molecular weight active substance and a stabilizer are uniformly mixed and formulated:

### (I)-1 Powders:

When the pharmaceutical composition of the present invention is powders, a low-molecular weight active substance and a stabilizer, aggregations of which are pulverized in advance with an oscillator, a pin mill, a hammer mill, etc., are mixed with an excipient being acceptable with regard to physical and chemical stability (lactose, mannitol, sorbitol, xylitol, trehalose, sucrose, erythritol, starch, crystalline cellulose, etc.) at a suitable ratio by using a V type mixer, a double-corn mixer, a ribbon type mixer, etc., if necessary, further thereto is added a lubricant such as talc, light anhydrous silicic acid, stearic acid or a salt thereof, sucrose fatty acid esters, etc. in order to protect from static electricity or improve the flow property, or in order to prevent the aggregation and caking of powder, to give a uniform powder. Further, if necessary, in order to retain the uniformity of the content of the low-molecular weight active substance, a low-molecular weight active substance and a part of an excipient, if necessary, and a part of a lubricant are added thereto to give a trituration with an oscillator, a pin mill, a hammer mill, etc., which is further mixed with a remaining additive to give the desired powders.

### (I)-2 Fine granules and granules:

When the pharmaceutical composition of the present invention is fine granules or granules (hereinafter, simply referred to as granules), for example, a low-molecular weight active substance and a stabilizer are mixed together with an excipient being acceptable with regard to physical and chemical stability (lactose, mannitol, sorbitol, xylictol, trehalose, sucrose, erythritol, starch, crystalline cellulose), binders (e.g., hydroxypropylcellulose, hydroxypropyl methylcellulose, pullulan, polyvinylpyrrolidone, etc.), if necessary, disintegrants (low-substituted hydroxypropylcellulose, carmellose calcium, croscarmellose sodium, cross-linked polyvinylpyrrolidone, carboxymethyl starch sodium, etc.), at a suitable rate by using a V type mixer, a double-corn type mixer, a ribbon type mixer, etc., if necessary, further thereto is added a glidant such as talc, light anhydrous silicic acid, etc. in order to improve the content uniformity and the flow property to give a uniform mixture. The mixture thus obtained is granulated by dry granulation method using a slug tablet machine, a compression roller, etc. or by wet granulation method using a molding granulator, a high-shear granulator or a fluid bed granulator and drier, then dried by a conventional method and regulated in size to give granules. Alternatively, the above-mentioned binder may be dissolved in advance in a solvent such as water, etc. to give a binder solution, which is added to a mixture of a low-molecular weight active substance, a stabilizer and an excipient as mentioned above, and the mixture is granulated, dried and regulated in size to give granules, by using a molding granulator, a high-shear granulator or a fluid bed granulator and drier. The obtained granules may be used as granules as they stand, but in order to protect from static electricity or improve the flow property thereof, talc, light anhydrous silicic acid, magnesium aluminometasilicate, stearic acid or a salt thereof, or sucrose fatty acid esters, etc. may be added as an anti-adhesion agent to the external of the granules. Alternatively, in the above methods for preparation of granule, the stabilizer of the present invention is not added to granules or fine granules (hereinafter, referred to as granules) but added to the external thereof together with an anti-adhesion agent, etc.

### (I)-3 Tablets and capsules:

When the pharmaceutical composition of the present invention is tablets or capsules, if necessary, an additive such as excipients, disintegrants, lubricants, etc. may be further added to the external of the above granules containing a low-molecular weight active substance (usually in an amount of 0.01 to 10 w/w % of the total weight of the pharmaceutical composition) and a stabilizer of the present invention at a suitable ratio, and the mixture is mixed and kneaded to give a powder for preparation. This powder for preparation is put into a capsule made of gelatin, hydroxypropyl methylcellulose or pullulan to give capsules. Further, this powder for preparation is compressed to give tablets.

In either case of capsules or tablets as mentioned above, the stabilizer of the present invention is not necessarily added to the granules but may be added to the external of the granules containing a low-molecular weight active substance.

Further, adversely, with respect to the granules containing a stabilizer (but not containing a low-molecular weight active substance), a low-molecular weight active substance and a suitable additive (excipient, disintegrant, lubricant, etc.) may be added to the external thereof, and the mixture is mixed to give a powder for preparation, which is put into a capsule to give capsules, or compressed to give tablets.

In addition, one of the stabilizer and the low-molecular weight active substance is previously formulated into granules (core particulate) or tablets (core tablet) together a substance being capable of supplying aldehyde-like substances, and then, the other one may be added thereto as a component consisting of a coating agent such as sugar coat, film coat or a component for capsules.

### (I)-4 Sustained-release granules, tablets and capsules:

Further, the granules or tablets containing a low-molecular weight active substance and a stabilizer as mentioned above but not containing a disintegrant are regarded as a core particulate or a core tablet, and a coating agent for controlling the drug dissolution which consists of a polymer, a fat and oil, etc., is coated thereto, and if necessary, and the resultant is further treated with heat (curing) to give sustained-release granules or tablets. Alternatively, the granules or tablets to which components for controlling the drug dissolution consisting of a polymer, a fat and oil, etc. are added but a disintegrant is not added are formulated by the above-mentioned method into granules or tablets, which are further treated with heat, if necessary, to give sustained-release granules or tablets. The granules thus obtained may be filled into a capsule to give capsules.

### (I)-5 Powdery injections:

When the pharmaceutical composition of the present invention is powdery injections, it may be prepared, for example, by putting a powder of a low-molecular weight active substance as aseptically prepared (including a lyophilized product) and a powder of a stabilizer of the present invention as aseptically prepared likewise (including a lyophilized product), and if necessary, together with an additive being conventionally used in the formulation procedures such as excipients (lactose, D-mannitol, D-sorbitol, glucose, sucrose, etc.) or pH adjustors, osmo-regulators, solubilizing agents, formulation stabilizers, etc. at a suitable ratio into an ampule, a vial, or other suitable vessels or kit vessels.

### (I)-6 Dry powder inhales:

When the pharmaceutical composition of the present invention is dry powder inhales, it may be prepared, for example, by putting a powder of a low-molecular weight active substance which is regulated into a suitable particle size for inhales and aseptically prepared (including a lyophilized product), and a powder of a stabilizer of the present invention as aseptically prepared likewise (including a lyophilized product), and if necessary, together with an additive being conventionally used in the formulation procedures such as excipients (lactose, D-mannitol, D-sorbitol, glucose, sucrose, etc.) or pH adjusting agents, osmo-regulators, solubilizing agents, formulation stabilizers, etc. at a suitable ratio into a suitable injector.

### (I)-7 Ointments and adhesive preparations:

When the pharmaceutical composition of the present invention is ointments or adhesive preparations, it may be prepared, for example, by dispersing a powder of a low-molecular weight active substance being regulated into a suitable particle size for external preparation, and a powder of a stabilizer of the present invention being regulated into a suitable particle size, and if necessary, together with pH adjusting agents, formulation stabilizers, absorption promoters, etc. which are conventionally used in the formulation procedures, at a suitable ratio into a base for ointment or adhesive preparation by using a suitable device in a conventional manner for ointment or adhesive preparation.

### (II) In cases where a low-molecular weight active substance-containing mass and a stabilizer-containing mass are separately prepared and formulated:

### (II)-1 Fine granules and granules:

When the pharmaceutical composition of the present invention is fine granules or granules, for example, a low-molecular weight active substance and a stabilizer are separately mixed with a substance being capable of supplying aldehyde-like substances, an excipient being acceptable with regard to physical and chemical stability (e.g., lactose, mannitol, sorbitol, xylitol, trehalose, sucrose, erythritol, starch, crystalline cellulose, sucrose, glucose, corn starch, licorice powder, sodium bicarbonate, calcium phosphate, calcium sulfate), binders (e.g., hydroxypropylcellulose, hydroxypropyl methylcellulose, pullulan, polyvinylpyrrolidone, gelatin, starch, gum arabic, tragacanth, carboxymethylcellulose, sodium arginate, glycerin), and if necessary, further disintegrants (e.g., low-substituted hyrdoxypropylcellulose, carmellose calcium, croscarmellose sodium, cross-linked polyvinylpyrrolidone, carboxymethyl starch sodium, an amino acid, starch, corn starch, calcium carbonate, crosspovidone) at a suitable ratio by using a V type mixer, a double-corn type mixer, a ribbon type mixer, etc., if necessary, further thereto is added a glidant such as talc, light anhydrous silicic acid, etc. in order to improve the content uniformity and the flow property to give a uniform mixture. The substance being capable of supplying aldehyde-like substances may be the same ones as excipients, binders, disintegrants, etc. being acceptable with regard to the physical and chemical stability, or may be different from them. The mixture thus obtained is granulated by dry granulation method using a slug tablet machine, a compression roller, etc. or by wet granulation method using a molding granulator, a high-shear granulator or a fluid bed granulator and drier, then if necessary, dried by a conventional method and regulated in size to give fine granules or granules. In the wet granulation method, the above-mentioned binder may be dissolved in advance in a solvent such as water, etc. to give a binder solution, and a low-molecular weight active substance or a stabilizer may be added to said binder solution.

In addition, by adhering (including partially or wholly coating) the above-mentioned mixture containing a low-molecular weight active substance or a stabilizer to a substantially spherical core such as Nonpareil (trade name; consisting of sucrose 75 % (w/w) and corn starch 25 % (w/w)), core-shell granules containing either a low-molecular weight active substance or a stabilizer are obtained.

The low-molecular weight active substance-containing granules (or fine granules) and the stabilizer-containing granules (or fine granules) thus obtained are uniformly mixed and used as granules (or fine granules). Moreover, in order to protect from static electricity or improve the flow property, talc, light anhydrous silicic acid, magnesium aluminometasilicate, stearic acid or a salt thereof (e.g., magnesium salt, calcium salt), or sucrose fatty acid esters may be added as an anti-adhesion agent to the external of the granules (or fine granules).

In addition, the above-mentioned mixture containing a low-molecular weight active substance or a stabilizer is successively adhered (including partially or wholly coating) or laminated onto a substantially spherical core such as Nonpareil (trade name; consisting of sucrose 75 % (w/w) and corn starch 25 % (w/w)), etc. to give core-shell granules containing a low-molecular weight active substance and a stabilizer.

Moreover, one of the mixtures containing a low-molecular weight active substance or a stabilizer is regarded as a core, and the other mixture is adhered (including partially or wholly coating) or laminated onto the core to give core-shell granules containing a low-molecular weight active substance and a stabilizer.

### (II)-2 Tablets and capsules:

When the pharmaceutical composition of the present invention is tablets or capsules, if necessary, an additive such as excipients, disintegrants, lubricants, etc. may be further added to the low-molecular weight active substance-containing granules (usually in an amount of 0.01 to 10 w/w % of the total weight of the pharmaceutical composition) and the stabilizer-containing granules as mentioned in the above (II)-1 at a suitable ratio, and the mixture is mixed to give a powder for preparation. This powder for preparation is put into a capsule made of gelatin, hydroxypropyl methylcellulose or pullulan to give capsules. Alternatively, this powder for preparation is compressed to give tablets. The low-molecular weight active substance-containing granules and the stabilizer-containing granules are uniformly mixed and then the mixture is compressed to give tablets where the low-molecular weight active substance-containing granules and the stabilizer-containing granules are uniformly dispersed within the preparation.

Further, tablets such as multilayer tablets or core-shell tablets may be obtained by individually setting the low-molecular weight active substance-containing granules and the stabilizer-containing granules in a discrete part of the preparation, followed by compressing the resultant. For instance, one of the low-molecular weight active substance-containing granules and the stabilizer-containing granules are tableted, if necessary, together with an additive (e.g., excipient, disintegrant, lubricant), and the other one is successively tableted by the same tableting punch to give the double-layer tablets. Alternatively, one of the low-molecular weight active substance-containing granules and the stabilizer-containing granules are tableted, if necessary, together with an additive (e.g., excipient, disintegrant, lubricant), and the obtained tablets are regarded as inner cores and applied with the other one as a component for shell to give the core-shell tablets.

In addition, the tablets may be obtained as well by using the core-shell granule containing a low-molecular weight active substance and a stabilizer in a similar manner to the above.

The fine granules, the granules, the tablets and the capsules obtained in the above (II)-1 and (II)-2 may be coated by a coating agent in order to give taste masking property, enteric property or sustained-release property.

The coating agent includes, for example, hydroxypropyl methylcellulose, ethylcellulose, hydroxymethylcelluose, hydroxypropylcellulose, polyoxyethyleneglycol, Tween 80, Pluronic F68, cellulose acetate phthalate, hydroxypropyl methylcellulose phthalate, hydroxymethylcellulose acetate succinate, methacrylic acid-acrylic acid copolymer (trade name: Eudragit, manufactured by Röhm & Haas GmbH, West Germany), and the like.

In order to improve the photostability of the low-molecular weight active substance, a light blocking agent such as titanium oxide, colcothar, etc. may be used in the coating step.

### (II)-3 Sustained-release granules, tablets and capsules:

Further, among the granules or tablets obtained by mixing the low-molecular weight active substance-containing granules and the stabilizer-containing granules and formulating the mixture into a suitable size by pulverization and the like, the granules or tablets to which a disintegrant is not added are regarded as a core particulate or core tablet, which are coated with a coating agent for controlling the drug dissolution consisting of a polymer, a fat and oil, etc., and if necessary, treated with heat (curing) to give the sustained-release granules or tablets. Alternatively, the sustained-release granules or tablets may be prepared by formulating the granules or the tablets to which a disintegrant is not added but a component for controlling the drug dissolution consisting of a polymer, a fat and oil, etc. is added, to the granules or tablets, if necessary, and further treating them with heat. The granules thus obtained may be put into capsules to give sustained-release capsules.

The present invention is illustrated in more detail by the following Experiments, Comparative Examples and Examples, but the present invention should not be construed to be limited thereto. Hereinafter, the compound expressed as Compound A means [3-[(2R)-[[(2R)-(3-chlorophenyl)-2-hydroxyethyl]amino]propyl]-1H-indol-7-yleoxy]acetic acid, which is an example for a low-molecular weight active substances of the present invention.

### Experiment 1: Confirmation of generation of aldehyde

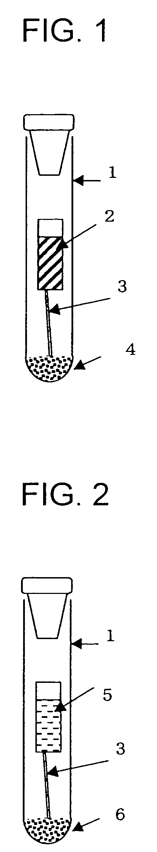

It was confirmed by the following experiment that aldehyde or aldehyde-like substances is generated from a pharmaceutical composition consisting of pharmaceutical additives being generally used or from a mixture of these additives, according to the storage conditions (temperature, storage period). Namely, a solid pharmaceutical composition (100 mg) being prepared by physically mixing components as listed in Experiment 1-1 or 1-2 of Table 1 was put into a stoppered glass test tube together with a cup containing a purified water for capture of aldehydes (1 ml) (see Fig. 1), and the test tube was stored at 40°C, 50°C and 60°C for 14 days, and the amount of formaldehyde captured in the purified water in the cup was measured. The warming procedure as mentioned above is an accelerated test for investigating the change with time of a sample for a short time, and the results thereof reflect changes when the sample is at an ambient temperature or room temperature for a long time.

The amount of formaldehyde in a sample was measured by reacting a sample with a 0.1 % solution (0.1 ml) of 2,4-dinitrophenylhydrazine (DNP) in 4N aqueous hydrochloric acid, neutralizing the resulting DNP derivative with a 4N sodium hydroxide, and injecting the mixture (20 µl) into high performance liquid chromatograph under the following conditions. Conditions for high performance liquid chromatograph:
Column: L-column ODS (inner diameter: 4.6 mm x 150 mm, manufactured by Judicial Foundation Chemicals Evaluation and Research Institute)
Mobile phase: Mixture of acetonitrile : water (1:1)
Detector: Ultraviolet-visible spectrophotometer (wave length for detection: 345 nm)
Temperature: Constant temperature around 40°C
Flow rate: 1 ml/min.

As a result, as shown in Experiment 1-1 in Table 2, since it was observed that the amount of formaldehyde to be captured in the cup was increased with the elevation of temperature for warming, it was confirmed that formaldehyde is generated by warming the solid pharmaceutical composition. This fact indicates that the solid pharmaceutical composition may become a resource of formaldehyde, and it is suggested that the solid pharmaceutical composition may provide conditions under which a related substance may be generated by reacting formaldehyde with a low-molecular weight active substance the stability of which is impaired by the effects of aldehydes.

**Table 1:**

| : Components of solid pharmaceutical composition | | |
|---|---|---|
| | Experiment 1-1 | Experiment 1-2 |
| Lactose (substance being capable of supplying aldehyde-like substances) | 71.0 parts | - parts |
| D-mannitol (substance being capable of supplying aldehyde-like substances) | - | 71.0 |
| Low-substituted hydroxypropylcellulose | 10.0 | 10.0 |
| Hydroxypropylcellulose | 2.5 | 2.5 |
| Crystalline cellulose | 15.0 | 15.0 |
| Magnesium stearate | 1.0 | 1.0 |
| Light anhydrous silicic acid | 0.5 | 0.5 |
| Total | 100.0 | 100.0 |
| [Note] In Table, "parts" means parts by weight, and hereinafter the same. | | |

**Table 2:**

| Amount of formaldehyde generated from the solid pharmaceutical composition of Experiment 1-1 or 1-2 | | |
|---|---|---|
| Storage condition | Captured amount of formaldehyde in the cup (µg) | |
| | Experiment 1-1 | Experiment 1-2 |
| At 40°C for 14 days | 0.0027 | 0.0030 |
| At 50°C for 14 days | 0.0129 | 0.0103 |
| At 60°C for 14 days | 0.0365 | 0.0291 |

### Experiment 2: Reaction of low-molecular weight active substance and aldehyde

When Compound A powder (2 mg) was stored.in a sealed stoppered glass test tube together with a cup containing an aqueous formaldehdye solution (1 ml) in various concentrations (see Fig. 2), and stored at 50°C for 14 days, the generated amount of related substances of Compound A was measured. The generated amount of the related substances was measured by dissolving Compound A powder (2 mg) in methanol (200 ml) to give a test solution, and applying 10 µl thereof to high performance liquid chromatograph under the following conditions. The percentage ratio of the peak area of the related substances to the total peak area including that of Compound A was regarded as the generation amount thereof.

### Conditions for high performance liquid chromatograph:

Column: Develosil ODS-5 (inner diameter: 4.6 mm x 150 mm, manufactured by NOMURA CHEMICAL CO., LTD.)
Mobile phase: Mixture of 0.01M citric acid buffer (pH 2.5) : acetonitrile (75:25)
Detector: Ultraviolet-visible spectrophotometer (wave length for detection: 220 nm)
Temperature: Constant temperature around 40°C
Flow rate: 1 ml/min.

As a result, as shown in Table 3, it was confirmed that the generated amount of related substances was increased in proportion to the concentration of formaldehyde in an aqueous solution. It indicates that formaldehyde vaporizing from the aqueous formaldehyde solution enclosed in the test tube diffuses into the air and reacts with Compound A powder.

**Table 3:**

| Relation between the formaldehyde concentration in the aqueous solution and the generated amount of related substances under coexistence of aqueous formaldehyde solution (after stored at 50°C for 14 days) | |
|---|---|
| Concentration of aqueous aldehyde solution in the cup (µg/ml) | Generated amount of related substances (%) |
| Not detected | Not detected |
| 0.0625 | Not detected |
| 0.125 | 0.031 |
| 0.25 | 0.063 |
| 0.5 | 0.091 |
| 1.0 | 0.136 |

### Experiment 3: Reaction of low-molecular weight active substance with aldehyde in pharmaceutical compositions

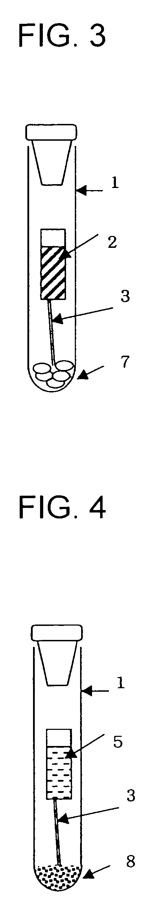

The following experiment was conducted in order to confirm that aldehyde is actually generated in tablets containing Compound A. Five tablets containing Compound A, which were prepared from the components of the formulation as shown in Table 4, Experiment 3-1 or Experiment 3-2, were put into a stoppered glass test tube together with a cup containing purified water (1 ml) for capturing formaldehyde (see Fig. 3), and the test tube was stored at 40°C, 50°C or 60°C for 28 days, and the generated amount of formaldehyde being captured in the purified water in the cup was measured in a similar manner to Experiment 1. The content of the related substances in the tablets was measured in such a manner that a sample was suspended in methanol (200 ml), and the mixture was centrifuged to collect a supernatant as a sample solution, which was further measured in a similar manner to Experiment 2.

As a result, as shown in Table 5, it was observed that there was am apparent correlation between the captured amount of aldehydes in the cup, and the generated amount of related substances. It indicates that aldehyde generated from tablets reacts with a low-molecular weight active substance to generate related substances.

**Table 4:**

| Components of the composition of formulation for solid preparation | | |
|---|---|---|
| | Experiment 3-1 | Experiment 3-2 |
| Compound A (low-molecular weight active substance) | 0.1 parts | 0.1 parts |
| Lactose (substance being capable of supplying aldehyde-like substances) | 70.9 | - |
| D-mannitol (substance being capable of supplying aldehyde-like substances) | - | 70.9 |
| Low-substituted hydroxypropylcellulose | 10.0 | 10.0 |
| Hydroxypropylcellulose | 2.5 | 2.5 |
| Crystalline cellulose | 15.0 | 15.0 |
| Magnesium stearate | 1.0 | 1.0 |
| Light anhydrous silicic acid | 0.5 | 0.5 |
| Total | 100.0 | 100.0 |

**Table 5:**

| Relation between the captured amount of formaldehyde and the generated amount of related substances | | | | |
|---|---|---|---|---|
| | Experiment 3-1 | | Experiment 3-2 | |
| Storage conditions | Captured amount of formaldehyde in the cup (µg) | Generated amount of related substance (%) | Captured amount of formaldehyde in the cup (µg) | Generated amount of related substance (%) |
| At the start | - | 0.14 | - | 0.22 |
| 40°C | 0.0512 | 0.52 | 0.0811 | 0.60 |
| 50°C | 0.4143 | 1.48 | 0.4827 | 1.55 |
| 60°C | 0.3435 | 7.97 | 0.3444 | 4.66 |

### Experiment 4: Absorption of aldehyde by stabilizer

Meglumine powder (1 g) was stored in a sealed stoppered glass test tube together with a cup containing an aqueous formaldehyde solution (2 ml) (containing potassium iodide for adjusting moisture) in various concentrations (see Fig. 4). For a comparative control, a cup containing an aqueous formaldehyde solution (2 ml) (containing potassium iodide for adjusting moisture) in various concentrations was put into a sealed stoppered glass test tube without meglumine. The test tubes were stored at 25°C or 40°C for 2 days, and the residual amount of formaldehyde in the cup was measured in a similar manner to Experiment 1.

As a result, as shown in Table 6, the residual ratio of formaldehyde in the cup after storage was apparently lowered by enclosing meglumine powder, as compared to the residual ratio of formaldehyde in the cup without enclosing meglumine powder. It indicates that meglumine powder can absorb the formaldehyde sublimated in the test tube from an aqueous formaldehyde solution in the cup.

**Table 6:**

| Residual rate (%) after storage at 25°C for 2 days | | | |
|---|---|---|---|
| Concentration of formaldehyde in the cup | 0.1 µg/ml | 0.5 µg/ml | 1 µg/ml |
| With enclosing meglumine | 85.1 | 75.5 | 70.7 |
| Without enclosing meglumine | 100 | 100 | 100 |

### Example 1, Example 2 and Comparative Example 1: Powders

The components of the formulation as shown in Example 1, Example 2 or Comparative Example 1 of Table 7 were weighed according to the relative proportion of each component, and mixed in a ceramic mortar to give powders containing Compound A.

The powders of Example 1, Example 2 or Comparative Example 1 were charged into the bottom of a stoppered glass test tube as shown in Fig. 3, and stored at 60°C for one week with sealing (relative humidity: 100 %). The generated amount of related substances was measured by high performance liquid chromatography in a similar manner to Experiment 3. The results thereof are shown in Table 8.

It was confirmed that the generated amount of the related substances in the powders containing DL-alanine or L-arginine of Example 1 and Example 2 was apparently lowered as compared to the powders containing no stabilizer of Comparative Example 1.

**Table 7**

| Component | Formulation | | |
|---|---|---|---|
| | Comparative Example 1 | Example 1 | Example 2 |
| Compound A (low-molecular weight active substance) | 0.1 parts | 0.1 parts | 0.1 parts |
| D-mannitol (substance being capable of supplying aldehyde-like substances) | 70.9 | 61.9 | 61.9 |
| DL-Alanine (stabilizer) | - | 10.0 | - |
| L-Arginine (stabilizer) | - | - | 10.0 |
| Magnesium stearate | 1.0 | 1.0 | 1.0 |
| Total | 72.0 parts | 73.0 parts | 73.0 parts |

**Table 8**

| Timing of measuring | Generated amount of related substances (%) | | |
|---|---|---|---|
| | Comparative Example 1 | Example 1 | Example 2 |
| At the start | Not detected | Not detected | Not detected |
| After one week-storage | 4.4 | 1.2 | 0.2 |

### Comparative Example 2 and Comparative Example 3: Tablets

Each component of the formulation as shown in Comparative Example 2 of Table 9 was weighed according to the relative proportion of each component, and Compound A, lactose and low-substituted hydroxypropylcellulose were granulated and dried by spraying thereto an aqueous solution of hydroxypropylcellulose (5 w/w %) in a fluid bed granulator and drier (FLO-2 type, manufactured by Freund Corporation), and regulated in size by using a stainless sieve (20 mesh) to give the low-molecular weight active substance-containing granules. To these low-molecular weight active substance-containing granules were added crystalline cellulose, magnesium stearate and light anhydrous silicic acid, and the mixture was mixed in a polyethylene bag to give granules for tableting. These granules for tableting were compressed by using a rotary tableting machine (HT-AP18SS-II type, manufactured by HATA IRON WORKS CO., LTD.) to give the tablets of Compound A.

Each component of the formulation as shown in Comparative Example 3 of Table 9 were weighed according the proportion ratio of each component, and the same procedures as the above were conducted to give the tablets of Compound A.

The tablets of Comparative Example 2 and Comparative Example 3 were put into a high-density polyethylene bottle, and stored at 40°C with 75 % RH for one month under sealing, or sealing with a silica gel enclosed, or opening, and the generated amount of related substances was measured by using high performance liquid chromatography in a similar manner to Experiment 3. The results are shown in Table 10.

It was confirmed that related substances were generated from the tablets of Comparative Example 2 (formulation using lactose) and Comparative Example 3 (formulation using mannitol) in a rate of 1 % or more.

**Table 9**

| Components | | Formulation | |
|---|---|---|---|
| | | Comparative Example 2 | Comparative Example 3 |
| Low-molecular weight active substance-containing granules | Compound A (low-molecular weight active substance) | 0.1 parts | 0.1 parts |
| | Lactose (substance being capable of supplying aldehyde-like substances) | 70.9 | - |
| | D-mannitol (substance being capable of supplying aldehyde-like substances) | - | 70.9 |
| | Low-substituted hydroxypropylcellulose | 10.0 | 10.0 |
| | Hydroxypropylcellulose | 2.5 | 2.5 |
| | Light anhydrous silicic acid | - | 0.2 |
| Components of post-addition | Crystalline cellulose | 15.0 parts | 15.0 parts |
| | Magnesium stearate | 1.0 | 1.0 |
| | Light anhydrous silicic acid | 0.5 | 0.3 |
| | Total | 100.0 parts | 100.0 parts |

**Table 10**

| Storage condition and timing of measuring | Generated amount (%) of related substances | |
|---|---|---|
| | Comparative Example 2 | Comparative Example 3 |
| At the start | 0.19 | 0.05 |
| After one month-storage under sealing | 0.20 | 0.25 |
| After one month-storage under sealing with silica gel enclosed | 0.12 | 0.17 |
| After one month-storage with opening | 1.54 | 1.41 |

### Example 3 and Example 4: Tablets

Each component of the formulation as shown in Example 3 of Table 11 was weighed according to the proportion ratio of each component, and to Compound A, D-mannitol and low-substituted hydroxypropylcellulose was added an aqueous hydrogenated gelatin solution (10 w/w %). The mixture was kneaded and granulated by using a universal mulling machine (5MD-type, manufactured by Shinagawa Machinery Works Co., Ltd.), and the resultant was dried at 50°C for 16 hours, and regulated in size by using a stainless sieve (20 mesh) to give low-molecular weight active substance-containing granules. To these low-molecular weight active substance-containing granules were added crystalline cellulose, magnesium stearate and light anhydrous silicic acid, and the mixture was mixed in a polyethylene bag to give the granules for tableting. These granules for tableting were compressed by using a single punch tableting machine (manufactured by Kikusui Seisakusho Ltd.) to give the tablets of Compound A.

Each component of the formulation as shown in Example 4 of Table 11 was weighed according to the proportion ratio of each component, and Compound A, D-mannitol, low-substituted hydroxypropylcellulose and light anhydrous silicic acid were granulated and dried by spraying thereto a solution of meglumine in an aqueous hydroxypropylcellulose solution (5 w/w %) in a fluid bed granulator and drier. The resultant was regulated in size by using a stainless sieve (20 mesh) to give low-molecular weight active substance-containing granules. To these low-molecular weight active substance-containing granules were added crystalline cellulose, magnesium stearate and light anhydrous silicic acid, and the mixture was mixed in a polyethylene bag to give the granules for tableting. These granules for tableting were compressed by a rotary tableting machine (HT-AP18SS-II type, manufactured by HATA IRON WORKS CO., LTD.) to give the tablets of Compound A.

The tablets of Example 3 and Example 4 were put into a high-density polyethylene bottle, and stored at 40°C with 75 % RH for one month under sealing, or sealing with a silica gel enclosed, or opening, and the generated amount of related substances was measured by using high performance liquid chromatography in a similar manner to Experiment 3. The results are shown in Table 12.

As compared to the tablets of Comparative Example 3 which are the same kind of tablets using mannitol, the amount of related substances generated from the tablets of Example 3 and Example 4, where gelatin or meglumine was added to the composition as a stabilizer, was significantly suppressed under either condition. Especially, when silica gel was enclosed together with the tablets, the inhibitory effect on the generation of the related substances was more excellent.

**Table 11**

| Components | | Formulation | |
|---|---|---|---|
| | | Example 3 | Example 4 |
| Low-molecular weight active substance-containing granules | Compound A (low-molecular weight active substance) | 0.1 parts | 0.1 parts |
| | D-mannitol (substance being capable of supplying aldehyde-like substances) | 70.9 | 69.9 |
| | Low-substituted hydroxypropylcellulose | 10.0 | 10.0 |
| | Light anhydrous silicic acid | - | 0.2 |
| | Hydroxypropylcellulose | - | 2.5 |
| | Gelatin (stabilizer) | 2.5 | - |
| | Meglumine (stabilizer) | - | 1.0 |
| Components for post-addition | Crystalline cellulose | 15.0 parts | 15.0 parts |
| | Magnesium stearate | 1.0 | 1.0 |
| | Light anhydrous silicic acid | 0.5 | 0.3 |
| | Total | 100.0 parts | 100.0 parts |

**Table 12**

| Storage condition and timing of measuring | Generated amount (%) of related substances | |
|---|---|---|
| | Example 3 | Example 4 |
| At the start | Not detected | Not detected |
| After one month-storage under sealing | 0.12 | Not detected |
| After one month-storage under sealing with silica gel enclosed | Not detected | Not detected |
| After one month-storage with opening | 0.63 | 0.33 |

### Example 5 and Example 6: Tablets

Each component for the formulation as shown Example 5 and Example 6 of Table 13 was weighed according to the relative proportion of each component, and Compound A, D-mannitol, low-substituted hydroxypropylcellulose and light anhydrous silicic acid were granulated and dried by spraying thereto a solution of L-arginine in an aqueous hydroxypropylcellulose solution (5 w/w %) in a fluid bed granulator and drier (FLO-2 type, manufactured by Freund Corporation), and the resultant was regulated in size by using a stainless sieve (20 mesh) to give the low-molecular weight active substance-containing granules. To these low-molecular weight active substance-containing granules were added crystalline cellulose, magnesium stearate and light anhydrous silicic acid, and the mixture was mixed in a polyethylene bag to give the granules for tableting. These granules for tableting were compressed by using a rotary tableting machine (HT-AP18SS-II type, manufactured by HATA IRON WORKS CO., LTD.) to give the tablets of Compound A.

The tablets of Example 5 and Example 6 were put into a high-density polyethylene bottle, and stored at 40°C with 75 % RH for one month under sealing, or sealing with a silica gel enclosed, or opening, and the generated amount of related substances was measured by using high performance liquid chromatography in a similar manner to Experiment 3. The results are shown in Table 14.

As compared to the tablets of Comparative Example 3 which are the same kind of tablets using mannitol, the amount of related substances generated from the tablets of Example 5 and Example 6, where L-arginine was added to the composition as a stabilizer, was significantly suppressed under either storage condition. The inhibitory effect on the generation of related substances was increased in proportion to the increase in the amount of L-arginine to be added (till 2 %).

**Table 13**

| Components | | Formulation | |
|---|---|---|---|
| | | Example 5 | Example 6 |
| Granules | Compound A (low-molecular weight active substance) | 0.1 parts | 0.1 parts |
| | D-mannitol (substance being capable of supplying aldehyde-like substances) | 75.7 | 74.7 |
| | Low-substituted hydroxypropylcellulose | 10.0 | 10.0 |
| | Light anhydrous silicic acid | 0.2 | 0.2 |
| | Hydroxypropylcellulose | 3.0 | 3.0 |
| | L-Arginine | 1.0 | 2.0 |
| Components for post-addition | Crystalline cellulose | 8.7 parts | 8.7 parts |
| | Magnesium stearate | 1.0 1.0 | 1.0 1.0 |
| | Light anhydrous silicic acid | 0.3 | 0.3 |
| | Total | 100.0 parts | 100.0 parts |

**Table 14**

| Storage condition and timing of measuring | Generated amount (%) of related substances | |
|---|---|---|
| | Example 5 | Example 6 |
| At the start | 0.03 | 0.14 |
| After one month-storage under sealing | 0.12 | 0.05 |
| After one month-storage under sealing with silica gel enclosed | 0.12 | 0.03 |
| After one month-storage with opening | 0.32 | 0.11 |

### Example 7: Tablets formulated by post-addition of stabilizer

Each component of the formulation as shown in Example 7 of Table 15 was weighed according to the relative proportion of each component, and Compound A, D-mannitol, low-substituted hydroxypropylcellulose and light anhydrous silicic acid were granulated and dried by spraying thereto a solution of L-arginine in an aqueous hydroxypropylcellulose solution (5 w/w %) in a fluid bed granulator and drier (FLO-2 type, manufactured by Freund Corporation), and the resultant was regulated in size by using a stainless sieve (20 mesh) to give the low-molecular weight active substance-containing granules. To these low-molecular weight active substance-containing granules were added meglumine, crystalline cellulose, magnesium stearate and light anhydrous silicic acid, and the mixture was mixed in a polyethylene bag to give the granules for tableting. These granules for tableting were compressed by using a rotary tableting machine (HT-AP18SS-II type, manufactured by HATA IRON WORKS CO., LTD.) to give the tablets of Compound A by meglumine post-addition method.

The tablets of Example 7 were put into a high-density polyethylene bottle, and stored at 40°C with 75 % RH for one month under sealing, or sealing with a silica gel enclosed, or opening, and the generated amount of related substances was measured by using high performance liquid chromatography in a similar manner to Experiment 3. The results are shown in Table 16.

When the formation where meglumine as a stabilizer does not directly contact with a low-molecular weight active substance, i.e., the tablets of Example 7, which were prepared by adding meglumine together with the components for post-addition into outside of the low-molecular weight active substance-containing granules and tableting, were stored at 40°C with 75 % RH for one month under sealing with silica gel enclosed, or opening, the sufficient stabilizing effect of low-molecular weight active substance was obtained, and there was obtained a stability being equal to the stability of the tablets of Example 4, which were prepared from the low-molecular weight active substance-containing granules granulated together with meglumine.

**Table 15**

| Components | | Example 7 |
|---|---|---|
| Granules | Compound A (low-molecular weight active substance) | 0.1 parts |
| | D-mannitol (substance being capable of supplying aldehyde-like substances) | 69.9 |
| | Low-substituted hydroxypropylcellulose | 10.0 |
| | Hydroxypropylcellulose | 2.5 |
| | Light anhydrous silicic acid | 0.2 |
| Components for post-addition | Meglumine (stabilizer) | 1.0 parts |
| | Crystalline cellulose | 15.0 |
| | Magnesium stearate | 1.0 |
| | Light anhydrous silicic acid | 0.3 |
| | Total | 100.0 parts |

**Table 16**

| Storage condition and timing of measuring | Generated amount (%) of related substances |
|---|---|
| | Example 7 |
| At the start | 0.16 |
| After one month-storage under sealing with silica gel enclosed | 0.20 |
| After one month-storage with opening | 0.32 |

### Example 8 and Example 9: Tablets formulated by post-addition of low-molecular weight active substance

Each component of the formulation as shown in Example 8 and Example 9 of Table 17 was weighed according to the relative proportion of each component, and D-mannitol and low-substituted hydroxypropylcellulose were granulated and dried by spraying thereto a solution of meglumine in an aqueous hydroxypropylcellulose solution (5 w/w %) in a fluid bed granulator and drier (FLO-5B type/15 type, manufactured by Freund Corporation), and the resultant was regulated in size by using a stainless sieve (20 mesh) to give the granules of meglumine. To these granules of meglumine were added Compound A, D-mannitol, crystalline cellulose, magnesium stearate and light anhydrous silicic acid, and the mixture was mixed in a polyethylene bag to give the granules for tableting. These granules for tableting were compressed by using a rotary tableting machine (Cleanpress Collect 19K-type, manufactured by Kikusui Seisakusho Ltd.) to give the tablets of Compound A by post-addition of low-molecular weight active substance.

The tablets of Example 8 and Example 9 were put into a high-density polyethylene bottle, and stored at 40°C with 75 % RH for one month under sealing, or sealing with a silica gel enclosed, or opening, and the generated amount of related substances was measured by using high performance liquid chromatography in a similar manner to Experiment 3. The results are shown in Table 18.

Either of the tablets of Example 8 or Example 9, which were prepared, unlike in the case of Example 7, by adding the low-molecular weight active substance together with the components of post-addition to outside of the meglumine granules without directly contacting said low-molecular weight active substance with meglumine of a stabilizer, a substance being capable of supplying aldehyde-like substances, showed an excellent stability under either condition, and the stability thereof was superior to that of the tablets of Example 4, which were prepared from the low-molecular weight active substance granulated together with meglumine. Further, in the case of the storage with silica gel enclosed, an extremely excellent inhibitory effect on the generation of related substances was obtained.

**Table 17**

| Components | | Formulation | |
|---|---|---|---|
| | | Example 8 | Example 9 |
| Granules | Meglumine (stabilizer) | 1.0 parts | 1.0 parts |
| | D-mannitol (substance being capable of supplying aldehyde-like substances) | 69.5 | 69.5 |
| | Low-substituted hydroxypropylcellulose | 10.0 | 10.0 |
| | Hydroxypropylcellulose | 2.5 | 2.5 |
| Components for post-addition | Compound A (low-molecular weight active substance) | 0.1 parts | 0.05 parts |
| | D-mannitol | 0.4 | 0.45 |
| | Crystalline cellulose | 15.0 | 15.0 |
| | Magnesium stearate | 1.0 | 1.0 |
| | Light anhydrous silicic acid | 0.5 | 0.5 |
| | Total | 100.0 parts | 100.0 parts |

**Table 18**

| Storage condition and timing of measuring | Generated amount (%) of related substances | |
|---|---|---|
| | Example 8 | Example 9 |
| At the start | Not detected | Not detected |
| After one month storage under sealing | Not detected | Not detected |
| After one month storage under sealing with silica gel enclosed | Not detected | Not detected |
| After one month storage with opening | 0.25 | 0.34 |

### Example 10 and Example 11: Tablets

Each component of the formulation as shown in Example 10 of Table 19 was weighed according to the relative proportion of each component, and hydralazine hydrochloride, lactose and low-substituted hydroxypropylcellulose and meglumine were granulated and dried by spraying thereto an aqueous solution of hydroxypropylcellulose (5 w/w %) in a fluid bed granulator and drier (FLO-5B type, manufactured by Freund Corporation), and the resultant was regulated in size by using a stainless sieve (20 mesh) to give the low-molecular weight active substance-containing granules. To these low-molecular weight active substance-containing granules were added crystalline cellulose, magnesium stearate and light anhydrous silicic acid, and the mixture was mixed in a polyethylene bag to give the granules for tableting. These granules for tableting were compressed by using a rotary tableting machine (HT-AP18SS-II type, manufactured by HATA IRON WORKS CO., LTD.) to give the stable tablets of hydralazine hydrochloride.

Each component of the formulation as shown in Example 11 of Table 19 was weighed according to the relative proportion of each component, and the same procedures as above were conducted to give the stable tablets of hydralazine hydrochloride.

**Table 19**

| | Components | Formulation | |
|---|---|---|---|
| | | Example 10 | Example 11 |
| Granules | Hydralazine hydrochloride (low-molecular weight active substance) | 5.0 parts | 10.0 parts |
| | Lactose (substance being capable of supplying aldehyde-like substances) | 61.0 | - |
| | D-mannitol (substance being capable of supplying aldehyde-like substances) | - | 56.0 |
| | Low-substituted hydroxypropylcellulose | 10.0 | 10.0 |
| | Hydroxypropylcellulose | 2.5 | 2.5 |
| | Meglumine (stabilizer) | 5.0 | 5.0 |
| | Light anhydrous silicic acid | - | 0.2 |
| Components for post-addition | Crystalline cellulose | 15.0 parts | 15.0 parts |
| | Magnesium stearate | 1.0 | 1.0 |
| | Light anhydrous silicic acid | 0.5 | 0.3 |
| | Total | 100.0 parts | 100.0 parts |

### Example 12 and Example 13: Tablets

Each component of the formulation as shown in Example 12 of Example 20 was weighed according to the relative proportion of each component, and to baclofen, D-mannitol and low-substituted hydroxypropylcellulose was added an aqueous solution of hydrogenated gelatin (10 w/w %). The mixture was kneaded and granulated by using a universal mulling machine (5MD-type, manufactured by Shinagawa Machinery Works Co., Ltd.), and the resultant was dried at 50°C for 16 hours, and regulated in size by using a stainless sieve (20 mesh) to give the low-molecular weight active substance-containing granules. To these low-molecular weight active substance-containing granules were added crystalline cellulose, magnesium stearate and light anhydrous silicic acid, and the mixture was mixed in a polyethylene bag to give the granules for tableting. These granules for tableting were compressed by using a single punch tableting machine (manufactured by Kikusui Seisakusho Ltd.) to give the stable tablets of baclofen.

Each component of the formulation as shown in Example 13 of Table 20 was weighed according to the relative proportion of each component, and the same procedures as the above were conducted to give the stable tablets of baclofen.

**Table 20**

| Components | | Formulation | |
|---|---|---|---|
| | | Example 12 | Example 13 |
| Granules | Baclofen (low-molecular weight active substance) | 2.0 parts | 2.0 parts |
| | D-mannitol (substance being capable of supplying aldehyde-like substances) | 67.0 | 67.0 |
| | Low-substituted hydroxypropylcellulose | 10.0 | 10.0 |
| | Light anhydrous silicic acid | - | 0.2 |
| | Hydroxypropylcellulose | - | 2.5 |
| | Gelatin (stabilizer) | 4.5 | - |
| | Meglumine (stabilizer) | - | 2.0 |
| Components for post-addition | Crystalline cellulose | 15.0 parts | 15.0 parts |
| | Magnesium stearate | 1.0 | 1.0 |
| | Light anhydrous silicic acid | 0.5 | 0.3 |
| | Total | 100.0 parts | 100.0 parts |

### Example 14 and Example 15: Tablets formulated by the post-addition of low-molecular weight active substance

Each component of the formulation as shown in Example 14 of Table 21 was weighed according to the relative proportion of each component, and D-mannitol and low-substituted hydroxypropylcellulose were granulated and dried by spraying thereto a solution of meglumine in an aqueous hydroxypropylcellulose solution (5 %) in a fluid bed granulator and drier (FLO-5B type/15 type, manufactured by Freund Corporation), and the resultant was regulated in size by using a stainless sieve (20 mesh) to give the granules of meglumine. To these granules of meglumine were added ephedrine hydrochloride, D-mannitol, crystalline cellulose, magnesium stearate and light anhydrous silicic acid, and the mixture was mixed in a polyethylene bag to give the granules for tableting. These granules for tableting were compressed by using a rotary tableting machine (Cleanpress Collect 19K-type, manufactured by Kikusui Seisakusho Ltd.) to give the stable tablets of ephedrine hydrochloride by post-addition of low-molecular weight active substance.

Each component of the formulation as shown in Example 15 of Table 21 was weighed according to the relative proportion of each component, and the same procedures as the above were conducted to give the stable tablets of ephedrine hydrochloride.

**Table 21**

| Components | | Formulation | |
|---|---|---|---|
| | | Example 14 | Example 15 |
| Granules | Meglumine (stabilizer) | 5.0 parts | 5.0 parts |
| | D-mannitol (substance being capable of supplying aldehyde-like substances) | 61.0 | 58.5 |
| | Low-substituted hydroxypropylcellulose | 10.0 | 10.0 |
| | Hydroxypropylcellulose | 2.5 | 2.5 |
| Components for post-addition | Ephedrine hydrochloride (low-molecular weight active substance) | 5.0 parts | 7.5 parts |
| | Crystalline cellulose | 15.0 | 15.0 |
| | Magnesium stearate | 1.0 | 1.0 |
| | Light anhydrous silicic acid | 0.5 | 0.5 |
| | Total | 100.0 parts | 100.0 parts |

### Example 16, Example 17 and Example 18

Each component of the formulation as shown in the premix powder for Example 16, Example 17 and Example 18 of Table 22 was dispersed by using a sample mill (AP-S type, screen diameter: 1 mm) to give the premix powder. This premix powder, D-mannitol, low-substituted hydroxypropylcellulose and light anhydrous silicic acid were put into a fluid bed granulator and drier (Flow Coater, FLO-5B type), and the mixture was granulated and dried by using an aqueous solution of hydroxypropylcellulose to give the low-molecular weight active substance-containing granules. Separately, mannitol and low-substituted hydroxypropylcellulose were put into a Flow Coaster (FLO-15 type); and the mixture was granulated and dried by using an aqueous solution of meglumine and hydroxypropylcellulose to give the stabilizer-containing granules. The low-molecular weight active substance-containing granules and the stabilizer-containing granules were separately sieved in a mill (twin roater equipped with 32 mesh sieve). The sieved low-molecular weight active substance-containing granules, the sieved stabilizer-containing granules, crystalline cellulose, magnesium stearate and light anhydrous silicic acid were mixed in a mixer (V-mixer (VM-10 type)) for 10 minutes to give the granules for tableting. These granules for tableting were compressed by using a rotary tableting machine (Cleanpress 19K) to give the tablets.

**Table 22**

| Components | | Formulation | | |
|---|---|---|---|---|
| | | Example 16 | Example 17 | Example 18 |
| Premix powder | Compound A (low-molecular weight active substance) | 0.1 parts | 0.2 parts | 0.3 parts |
| | D-mannitol (substance being capable of supplying aldehyde-like substances) | 0.86 | 1.72 | 2.58 |
| | Light anhydrous silicic acid | 0.04 | 0.08 | 0.12 |
| | Subtotal (premix powder) | 1 | 2 | 3 |
| Low-molecular weight active substance-containing granules | Premix powder | 1 | 2 | 3 |
| | D-mannitol (substance being capable of supplying aldehyde-like substances) | 35.59 | 34.63 | 33.67 |
| | Low-substituted hydroxypropylcell ulose | 5 | 5 | 5 |
| | Hydroxypropylcell ulose | 0.25 | 1.25 | 1.25 |
| | Light anhydrous silicic acid | 0.16 | 0.12 | 0.08 |
| | Purified water | 23.75 | 23.75 | 23.75 |
| | Subtotal (low-molecular weight active substance-containing granules) | 43 | 43 | 43 |
| Stabilizer-containing granules | D-mannitol (substance being capable of supplying aldehyde-like substances) | 33.45 | 33.45 | 33.45 |
| | Low-substituted hydroxypropylcell ulose | 5 | 5 | 5 |
| | Hydroxypropylcell ulose | 1.25 | 1.25 | 1.25 |
| | Meglumine (stabilizer) | 1 | 1 | 1 |
| | Purified water | 23.75 | 23.75 | 23.75 |
| | Subtotal (stabilizer-containing granules) | 40.7 | 40.7 | 40.7 |
| Low-molecular weight active substance-containing granules | | 43 | 43 | 43 |
| Stabilizer-containing granules | | 40.7 | 40.7 | 40.7 |
| Crystalline cellulose | | 15 | 15 | 15 |
| Magnesium stearate | | 1 | 1 | 1 |
| Light anhydrous silicic acid | | 0.3 | 0.3 | 0.3 |
| Total | | 100 parts | 100 parts | 100 parts |

### Example 19

Hydroxypropylcellulose (62.5 g) was dissolved in purified water (1188 g) to give Binding Liquid 1. Compound A (5.0 g), mannitol (1783 g) and low-substituted hydroxypropylcellulose (250 g) were uniformly mixed in a fluid bed granulator and drier (FD-3S, manufactured by Fuji Sangyo Ltd.), and then the mixture was granulated by spraying Binding Liquid 1 thereto in said apparatus. The obtained granules were dried in the fluid bed granulator and drier to give the granules of the drug substance.

Separately, hydroxypropylcellulose (62.5 g) and meglumine (50.0 g) were dissolved in purified water (1188 g) to give Binding Liquid 2. In a fluid bed granulator and drier (FD-3S, manufactured by Powrex Corporation), light anhydrous silicic acid (25.0 g, AEROSIL; NIPPON AEROSIL CO., LTD.), mannitol (1713 g) and low-substituted hydroxypropylcellulose (250 g) were uniformly mixed, and granulated by spraying thereto Binding Liquid 2 in said apparatus. The resultant was dried in the fluid bed granulator and drier to give the granules of meglumine.

Two kinds of the granules thus obtained were pulverized by using a power mill pulverizer (P-3, Showa Kagaku Kikai Kosakusho), with a punching screen (1.5 mm Φ) to give the pulverized powder.

The drug substance-containing pulverized powder (1680 g) and the meglumine-containing pulverized powder (1680 g) thus obtained, and microcrystalline cellulose (600 g) and magnesium stearate (40.0 g) were added, and mixed in a tumbler mixer (TM-60S, Showa Kagaku Kikai Kosakusho) to give the granules for tableting. The granules thus obtained were tableted by using a rotary tableting machine (Collect 19K, manufactured by Kikusui Seisakusho Ltd.) with a punch (6.5 mmΦ) under a tableting pressure of 7KN/punch to give the tablets (weight; 100 mg) of the following formulation containing 0.1 mg of Compound A per tablet.

| Formulation (components/tablet): | |
|---|---|
| 1) Compound A | 0.1 mg |
| 2) Mannitol | 69.9 mg |
| 3) Meglumine | 1.0 mg |
| 4) Hydroxypropylcellulose | 2.5 mg |
| 5) Low-substituted hydroxypropylcellulose | 10.0 mg |
| 6) Light anhydrous silicic acid | 0.5 mg |
| 7) Microcrystalline cellulose | 15.0 mg |
| 8) Magnesium stearate | 1.0 mg |
| Total | 100.0 mg |

### Example 20

Hydroxypropylcellulose (112.5 g) was dissolved in purified water (2138 g) to give Binding Liquid 1. In a fluid bed granulator and drier (FD-5S, Powrex Corporation), Compound A (9.0 g), mannitol (3209 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules of the drug substance.

Separately, hydroxypropylcellulose (112.5 g) and meglumine (90.0 g) were dissolved in purified water (2138 g) to give Binding Liquid 2. In a fluid bed granulator and drier (FD-5S, Powrex Corporation), mannitol (3128 g) and low-substituted hydroxypropylcellulose (450 g) were uniformly mixed, and granulated by spraying thereto Binding Liquid 2 in said apparatus. The resultant was dried in the fluid bed granulator and drier to give the granules of meglumine.

Two kinds of the granules thus obtained were pulverized by using a power mill pulverizer (P-3, Showa Kagaku Kikai Kosakusho), and milled with a punching screen (1.5 mm Φ) to give the pulverized powder.

The drug substance-containing pulverized powder (3486 g) and the meglumine-containing pulverized powder (3486 g) thus obtained, and microcrystalline cellulose (1245 g) and magnesium stearate (83.0. g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (6.5 mmΦ) under a tableting pressure of 6KN/punch to give the tablets (weight; 100 mg) of the following formulation containing 0.1 mg of Compound A per tablet.

| Formulation (components/tablet): | |
|---|---|
| 1) Compound A | 0.1 mg |
| 2) Mannitol | 70.4 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| Total | 100.0 mg |

### Example 21

Hydroxypropylcellulose (112.5 g) was dissolved in purified water (2138 g) to give Binding Liquid 1. In a fluid bed granulator and drier (FD-5S, Powrex Corporation), Compound A (18.0 g), mannitol (3200 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules containing the drug substance.

Separately, hydroxypropylcellulose (112.5 g) and meglumine (90.0 g) were dissolved in purified water (2138 g) to give Binding Liquid 2. In a fluid bed granulator and drier (FD-5S, Powrex Corporation), mannitol (3128 g) and low-substituted hydroxypropylcellulose (450 g) were uniformly mixed, and granulated by spraying thereto Binding Liquid 2 in said apparatus. The resultant was dried in the fluid bed granulator and drier to give the granules containing meglumine.

Two kinds of the granules thus obtained were pulverized by using a power mill pulverizer (P-3, Showa Kagaku Kikai Kosakusho), and milled with a punching screen (1.5 mm Φ) to give the pulverized powder.

The drug substance-containing pulverized powder (3486 g) and the meglumine-containing pulverized powder (3486 g) thus obtained, and microcrystalline cellulose (1245 g) and magnesium stearate (83.0 g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (6.5 mmΦ) under a tableting pressure of 6KN/punch to give the tablets (weight; 100 mg) of the following formulation containing 0.2 mg of Compound A per tablet.

| Formulation (components/tablet): | |
|---|---|
| 1) Compound A | 0.2 mg |
| 2) Mannitol | 70.3 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| Total | 100.0 mg |

### Example 22

Hydroxypropylcellulose (112.5 g) was dissolved in purified water (2138 g) to give Binding Liquid 1. In a fluid bed granulator and drier (FD-5S, Powrex Corporation), Compound A (27.0 g), mannitol (3191 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules containing the drug substance.

Separately, hydroxypropylcellulose (112.5 g) and meglumine (90.0 g) were dissolved in purified water (2138 g) to give Binding Liquid 2. In a fluid bed granulator and drier (FD-5S, Powrex Corporation), mannitol (3128 g) and low-substituted hydroxypropylcellulose (450 g) were uniformly mixed, and granulated by spraying thereto Binding Liquid 2 in said apparatus. The resultant was dried in the fluid bed granulator and drier to give the granules containing meglumine.

Two kinds of the granules thus obtained were pulverized by using a power mill pulverizer (P-3, Showa Kagaku Kikai Kosakusho), and milled with a punching screen (1.5 mm Φ) to give the pulverized powder.

The drug substance-containing pulverized powder (3486 g) and the meglumine-containing pulverized powder (3486 g) thus obtained, and microcrystalline cellulose (1245 g) and magnesium stearate (83 g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (6.5 mmΦ) under a tableting pressure of 6KN/punch to give the tablets (weight; 100 mg) of the following formulation containing 0.3 mg of Compound A per tablet.

| Formulation (components/tablet): | |
|---|---|
| 1) Compound A | 0.3 mg |
| 2) Mannitol | 70.2 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| Total | 100.0 mg |

### Example 23

Hydroxypropylcellulose (112.5 g) was dissolved in purified water (2138 g) to give Binding Liquid 1. In a fluid bed granulator and drier (FD-5S, Powrex Corporation), Compound A (9.0 g), mannitol (3209 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules containing the drug substance.

Separately, hydroxypropylcellulose (112.5 g) and meglumine (90.0 g) were dissolved in purified water (2138 g) to give Binding Liquid 2. In a fluid bed granulator and drier (FD-5S, Powrex Corporation), mannitol (3128 g) and low-substituted hydroxypropylcellulose (450 g) were uniformly mixed, and granulated by spraying thereto Binding Liquid 2 in said apparatus. The resultant was dried in the fluid bed granulator and drier to give the granules containing meglumine.

Two kinds of the granules thus obtained were pulverized by using a power mill pulverizer (P-3, Showa Kagaku Kikai Kosakusho), and milled with a punching screen (1.5 mm Φ) to give the pulverized powder.

The drug substance-containing pulverized powder (3486 g) and the meglumine-containing pulverized powder (3486 g) thus obtained, and microcrystalline cellulose (1245 g) and magnesium stearate (83.0 g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (major axis: 8.5 mm; minor axis: 5 mm) under a tableting pressure of 4KN/punch to give the tablets (weight; 100 mg) of the following formulation containing 0.1 mg of Compound A per tablet.

| Formulation (components/tablet): | |
|---|---|
| 1) Compound A | 0.1 mg |
| 2) Mannitol | 70.4 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| Total | 100.0 mg |

### Example 24

Hydroxypropylcellulose (112.5 g) was dissolved in purified water (1413 g) to give Hyroxypropylcellulose Liquid 1. Yellow ferric oxide (4.5 g) was dispersed in purified water (630 g) by using a Labodisper (ChuoRika), and purified water (90.0 g) and Hyroxypropylcellulose Liquid 1 were mixed to give Binding Liquid 1. In a fluid bed granulator and drier (FD-5S, Powrex Corporation), Compound A (18.0 g), mannitol (3195 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules containing the drug substance.

Separately, hydroxypropylcellulose (112.5 g) was dissolved in purified water (1323 g) to give Hyroxypropylcellulose Liquid 2. Next, yellow ferric oxide (4.5 g) was dispersed in purified water (630 g) by using a Labodisper (ChuoRika), and meglumine (90 g), purified water (90 g) and Hyroxypropylcellulose Liquid 2 were mixed to give Binding Liquid 2. In a fluid bed granulator and drier (FD-5S, Powrex Corporation), mannitol (3123 g) and low-substituted hydroxypropylcellulose (450 g) were uniformly mixed, and granulated by spraying thereto Binding Liquid 2 in said apparatus. The resultant was dried in the fluid bed granulator and drier to give the granules containing meglumine.

Two kinds of the granules thus obtained were pulverized by using a power mill pulverizer (P-3, Showa Kagaku Kikai Kosakusho), and milled with a punching screen (1.5 mm Φ) to give the pulverized powders.

The drug substance-containing pulverized powder (3486 g) and the meglumine-containing pulverized powder (3486 g) thus obtained, and microcrystalline cellulose (1245 g) and magnesium stearate (83.0 g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (major axis: 8.5 mm; minor axis: 5 mm) under a tableting pressure of 4KN/punch to give the tablets (weight; 100 mg) of the following formulation containing 0.2 mg of Compound A per tablet.

| Formulation (components/tablet): | |
|---|---|
| 1) Compound A | 0.2 mg |
| 2) Mannitol | 70.2 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| 8) Yellow ferric oxide | 0.1 mg |
| Total | 100.0 mg |

### Example 25

Hydroxypropylcellulose (112.5 g) was dissolved in purified water (1415 g) to give Hyroxypropylcellulose Liquid 1. Yellow ferric oxide (2.25 g) was dispersed in purified water (630 g) by using a Labodisper (ChuoRika), and purified water (90 g) and Hyroxypropylcellulose Liquid 1 were mixed to give Binding Liquid 1. In a fluid bed granulator and drier (FD-5S, Powrex Corporation), Compound A (27.0 g), mannitol (3188 g) and low-substituted hydroxypropylcellulose (450 g) were uniformly mixed and the mixture was granulated by spraying thereto Binding Liquid 1 in said apparatus. The obtained granules were dried in the fluid bed granulator and drier to give the granules containing the drug substance.

Separately, hydroxypropylcellulose (112.5 g) was dissolved in purified water (1325 g) to give Hyroxypropylcellulose Liquid 2. Next, yellow ferric oxide (2.25 g) was dispersed in purified water (630 g) by using a Labodisper (ChuoRika), and meglumine (90.0 g), purified water (90.0g) and Hyroxypropylcellulose Liquid 2 were mixed to give Binding Liquid 2. In a fluid bed granulator and drier (FD-5S, Powrex Corporation), mannitol (3125 g) and low-substituted hydroxypropylcellulose (450 g) were uniformly mixed, and granulated by spraying thereto Binding Liquid 2 in said apparatus. The resultant was dried in the fluid bed granulator and drier to give the granules containing meglumine.

Two kinds of the granules thus obtained were pulverized by using a power mill pulverizer (P-3, Showa Kagaku Kikai Kosakusho), and milled with a punching screen (1.5 mm Φ) to give the pulverized powders.

The drug substance-containing pulverized powder (3486 g) and the meglumine-containing pulverized powder (3486 g) thus obtained, and microcrystalline cellulose (1245 g) and magnesium stearate (83.0 g) were added and mixed in a tumbler mixer (TM-60S, Showa Kagaku Kikai Kosakusho) to give the granules for tableting. The granules thus obtained were compressed by using a rotary tableting machine (Collect 12HUK, Kikusui Seisakusho Ltd.) with a punch (major axis: 8.5 mm; minor axis: 5 mm) under a tableting pressure of 4KN/punch to give the tablets (weight; 100 mg) of the following formulation containing 0.3 mg of Compound A per tablet.

| Formulation (components/tablet): | |
|---|---|
| 1) Compound A | 0.3 mg |
| 2) Mannitol | 70.1 mg |
| 3) Meglumine | 1.0 mg |
| 4) Hydroxypropylcellulose | 2.5 mg |
| 5) Low-substituted hydroxypropylcellulose | 10.0 mg |
| 6) Microcrystalline cellulose | 15.0 mg |
| 7) Magnesium stearate | 1.0 mg |
| 8) Yellow ferric oxide | 0.1 mg |
| Total | 100.0 mg |

### INDUSTRIALLY APPLICABILITY

The composition of the present invention may stably retain the activity of a low-molecular weight active substance in the compositions containing a substance being capable of supplying aldehyde-like substances for medicaments, cosmetics, hair care products, etc., by containing a low-molecular weight active substance the stability of which is impaired by the effects of aldehydes, and a stabilizer having an amine structure and being capable of absorbing an aldehyde.

## Claims

1. A composition containing a substance being capable of supplying aldehyde-like substances, which further comprises a low-molecular weight active substance the stability of which is impaired by the effects of aldehydes, and a stabilizer having an amine structure and being capable of absorbing aldehydes.

2. The composition according to claim 1, wherein the stabilizer is an aminosugar or a polymer thereof, an aminosugar alcohol or a polymer thereof, an amino acid or a polymer thereof, a protein or a hydrolysate thereof, an alkylamine, a hydroxyalkylamine, or a salt thereof.

3. The composition according to claim 2, wherein the stabilizer is chitin, chitosan, chitooligosaccharide, meglumine, alanine, arginine, lysine, hydroxylysine, gelatin or a hydrolysate thereof, collagen or a hydrolysate thereof, albumin or a hydrolysate thereof, casein or a hydrolysate thereof, protamine or a hydrolysate thereof, diethylamine, hexylamine, tris(hydroxymethyl)aminomethane, or a salt thereof.

4. The composition according to claim 3, wherein the stabilizer is meglumine, L-arginine, gelatin, or a salt thereof.

5. The composition according to any one of claims 1-4, which is a pharmaceutical composition containing a low-molecular weight active substance and a stabilizer both in the form of a solid powder.

6. The composition according to claim 5, which is a pharmaceutical composition of solid form or semisolid form.

7. The composition according to claim 6, which is the solid or semisolid pharmaceutical composition selected from powders, fine granules, granules, tablets, capsules, powdery injections, dry powder inhales, ointments, and adhesive preparations.

8. The composition according to claim 1, which is prepared by uniformly mixing a low-molecular weight active substance and a stabilizer.

9. The composition according to claim 1, which is prepared by previously granulating one of a low-molecular weight active substance and a stabilizer together with a substance being capable of supplying aldehyde-like substances, followed by uniformly mixing the resultant with the other.

10. The composition according to claim 9, which is prepared by previously granulating a stabilizer together with a substance being capable of supplying aldehyde-like substances, followed by uniformly mixing the resultant with a low-molecular weight active substance so that the contact between the substance being capable of supplying aldehyde-like substances and the low-molecular weight active substance is prevented or lessened.

11. A pharmaceutical composition, which comprises a mass containing a low-molecular weight active substance the stability of which is impaired by the effects of aldehydes, and a mass containing a stabilizer having an amine structure and being capable of absorbing aldehydes, and at least one of these masses contains a substance being capable of supplying aldehyde-like substances.

12. The composition according to claim 11, wherein both of the mass containing a low-molecular weight active substance and the mass containing a stabilizer are in the form of a granule.

13. The composition according to claim 11, wherein both of the mass containing a low-molecular weight active substance and the mass containing a stabilizer are in the form of a fine granule.

14. The composition according to claim 11, which is in the form of a capsule prepared by filling granules and/or fine granules containing a low-molecular weight active substance, and granules and/or fine granules containing a stabilizer into capsules.

15. The composition according to claim 11, which in the form of a tablet prepared by tableting granules and/or fine granules containing a low-molecular weight active substance, and granules and/or fine granules containing a stabilizer.

16. A method of stabilizing a low-molecular weight active substance the stability of which is impaired by the effects of aldehydes in a composition containing a substance supplying aldehyde-like substances, which comprises adding a stabilizer having an amine structure and being capable of absorbing aldehydes when mixing said low-molecular weight active substance the stability of which is impaired by the effects of aldehydes.

17. The stabilization method according to claim 16, which comprises uniformly mixing a substance supplying aldehyde-like substances, a low-molecular weight active substance the stability of which is impaired by the effects of aldehydes and a stabilizer having an amine structure and being capable of absorbing an aldehyde.

18. The stabilization method according to claim 16, which comprises previously granulating one of a low-molecular weight active substance the stability of which is impaired by the effects of aldehydes and a stabilizer having an amine structure and being capable of absorbing aldehydes together with a substance supplying aldehyde-like substances, followed by mixing the resultant with the other.

19. The stabilization method according to claim 18, which comprises previously granulating a stabilizer having an amine structure and being capable of absorbing aldehydes together with a substance supplying aldehyde-like substances, followed by mixing the resultant with a low-molecular weight active substance the stability of which is impaired by the effects of aldehydes.

20. The stabilization method according to claim 16, which comprises preparing a mass containing a low-molecular weight active substance the stability of which is impaired by the effects of aldehydes and a mass containing a stabilizer having an amine structure and being capable of absorbing aldehydes separately, during which a substance supplying aldehyde-like substances is contained in one or both of these messes, followed by combining and mixing these two masses.
